# FASCICULE DE BREVET EUROPEEN

(11) **EP 1 323 413 B1**
(45) Date de publication et mention de la délivrance du brevet: **21.12.2016**
(21) Numéro de dépôt: 02292839.4
(22) Date de dépôt: 14.11.2002
(51) Int. Cl.: A61K 8/35, A61K 8/44, A61K 8/49, A61Q 17/04

(54) **Composition filtrante contenant un dérivé de 1,3,5-triazine, un dérivé du dibenzoylméthane, et un dérivé de 2-hydroxybenzophénone aminosubstitué**
Ein 1,3,5-Triazin-Derivat, ein Dibenzoylmethan-Derivat und ein amino-substituiertes 2-Hydroxy-benzophenon-Derivat enthaltende Lichtschutzmittelzusammensetzung
Light screening composition comprising a 1,3,5-triazine derivative, a dibenzoylmethane derivative and an amino substituted 2-hydroxybenzophenone derivative

(30) Priorité: 07.12.2001 FR 0115859
(43) Date de publication de la demande: 02.07.2003
(73) Titulaire: L'Oréal, 75008 Paris (FR)
(72) Inventeur: Candau, Didier, 91570 Bièvres (FR)
(74) Mandataire: Casalonga

(56) Documents cités:
- EP-A- 1 046 391
- EP-A- 1 133 980
- WO-A-01/85123
- PATENT ABSTRACTS OF JAPAN vol. 2000, no. 01, 31 janvier 2000 (2000-01-31) & JP 11 292748 A (26-101999)

## Description

L'invention se rapporte à l'utilisation d'un dérivé de 2-hydroxybenzophénone aminosubstitue particulier pour la fabrication d'une composition cosmétique ou dermatologique, à usage topique, en particulier pour la photoprotection de la peau et des cheveux, qui comprend, dans un support cosmétiquement acceptable :
(a) au moins un dérivé du dibenzoytméthane et
(b) au moins un dérivé de 1,3,5-triazine particulier photosensible en présence d'un dérivé du dibenzoytméthane et
(c) au moins ledit dérivé de 2-hydroxybenzophénone aminosubstitué particulier ; ladite composition ne contenant pas de p-methytbenzytidènecamphre,
en vue d'améliorer dans ladite composition la stabilité au rayonnement UV dudit dérivé de 1,3,5-triazine.

On sait que les radiations lumineuses de longueurs d'onde comprises entre 280 nm et 400 nm permettent le brunissement de l'épiderme humain, et que les rayons de longueurs d'onde plus particulièrement comprises entre 280 et 320 nm, connus sous la dénomination UV-B, provoquent des érythèmes et des brûlures cutanées qui peuvent nuire au développement du bronzage naturel. Pour ces raisons ainsi que pour des raisons esthétiques, il existe une demande constante de moyens de contrôle de ce bronzage naturel en vue de contrôler ainsi la couleur de la peau ; il convient donc de filtrer ce rayonnement UV-B.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement cutané prématuré. Ils favorisent le déclenchement de la réaction érythémateuse ou amplifient cette réaction chez certains sujets et peuvent même être à l'origine de réactions phototoxiques ou photo-allergiques. Ainsi, pour des raisons esthétiques et cosmétiques telles que la conservation de l'élasticité naturelle de la peau par exemple, de plus en plus de gens désirent contrôler l'effet des rayons UV-A sur leur peau. Il est donc souhaitable de filtrer aussi le rayonnement UV-A.

A cet égard, une famille de filtres UV-A particulièrement intéressante est actuellement constituée par les dérivés du dibenzoylméthane, est notamment le 4-ter-butyl-4'-méthoxydibenzoyl méthane, qui présentent en effet un fort pouvoir d'absorption intrinsèque. Ces dérivés du dibenzoylméthane, qui sont maintenant des produits bien connus en soi à titre de filtres actifs dans les UV-A, sont notamment décrits dans les demandes de brevets français FR-A-2326405 et FR-A-2440933, ainsi que dans la demande de brevet européen EP-A-0114607 ; le 4-ter-butyl- 4'-méthoxydibenzoyl méthane est par ailleurs actuellement proposé à la vente sous la dénomination commerciale de "PARSOL 1789" par la société HOFFMANN LAROCHE.

Les dérivés de 1,3,5-triazine sont particulièrement recherchés dans la cosmétique solaire du fait qu'ifs sont fortement actifs dans l' UVB et même dans l'UV-A pour certains de ces composés selon la nature des substituants mis en jeu. Ils sont notamment décrits dans les demandes de brevet US 4367390, EP863145, EP517104, EP570838, EP507691, EP796851, EP775698, EP878469 et EP933376, et on connaît en particulier :
- la 2,4,6-tris[p-(2'-éthylhexyl-1'-oxycarbonyl)anilino]-1,3,5-triazine ou « Ethylhexyl Triazone» (nom INCI), vendue sous la dénomination commerciale « Uvinul T 150 » par la société BASF,
- la 2-[p-(tertiobutylamido)anilino]-4,6-bis-[(p-(2'-éthylhexyl-1'-oxycarbonyl)anilino]-1,3,5-triazine ou « Diethylhexyl Butamido Triazone » (nom INCI) vendu sous le nom commercial « UVASORB HEB » par SIGMA 3V. Ils possèdent un fort pouvoir absorbant des UVB et il serait donc très intéressant de pouvoir les utiliser en association avec le 4-tert-butyl-4'-méthoxydibenzoylméthane cité ci-dessus dans le but d'obtenir des produits offrant une protection large et efficace dans l'ensemble du rayonnement UV.

On connaît dans les demandes de brevet DE 100 12 408 et EP1046391 des compositions solaires de dérivés de 2-hydroxybenzophénone aminosubstitués pouvant contenir d'autres filtres complémentaires comme les dérivés de dibenzoylméthane et les dérivés de triazine tels que mentionnés ci-dessus en présence de p-méthylbenzylidène camphre.

Toutefois, la Demanderesse a constaté que certains de ces dérivés de 1,3,5-triazine, lorsqu'ils sont en présence de 4-tert-butyl-4'-méthoxydibenzoylméthane sont photosensibles à savoir : sous irradiation UV, ils présentent l'inconvénient de se dégrader chimiquement de façon importante. Dans ces conditions, l'association des deux filtres ne permet plus une protection solaire large prolongée de la peau et des cheveux.

Or, à la suite d'importantes recherches menées dans le domaine de la photoprotection évoqué ci-dessus, la Demanderesse a maintenant découvert que l'introduction d'un dérivé de 2-hydroxybenzophénone aminosubstitué particulier dans une composition contenant du 4-tert-buty)-4'-méthoxydibenzoylméthane en association avec au moins un dérivé de 1,3,5-triazine photosensible en présence dudit dibenzoylméthane, permettait d'améliorer de façon tout à fait remarquable la photostabilité de ce dérivé de 1,3,5-triazine, au sein de telles compositions, et donc l'efficacité globale de ces compositions.

Cette découverte est à la base de l'invention.

La présente invention a donc pour objet l'utilisation d'un dérivé de 2-hydroxybenzophénone aminosubstitué de formule (VIII) suivante : 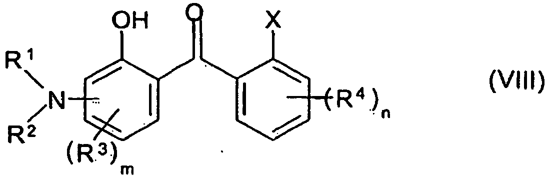 dans laquelle :
R¹ et R , identiques ou différents, désignent un atome d'hydrogène, un radical alkyle en C₁-C₂₀, un radical alcènyle en C₂-C₁₀, un radical cycloalkyle en C₃-C₁₀, un radical cycloalcènyle en C₃-C₁₀ ;
R¹ et R² peuvent également former avec l'atome d'azote avec lequel ils sont liés un cycle à 5 ou 6 chaînons ;
R³ et R⁴, identiques ou différents, désignent un radical alkyle en C₁-C₂₀, un radical alcènyle en C₂-C₁₀, un radical cycloalkyle en C₃-C₁₀, un radical cycloalcènyle en C₃-C₁₀, un radical alcoxy en C₁-C₁₂, un radical (C₁-C₂₀)alcoxycarbonyle, un radical alkylamino en C₁-C₁₂, un radical dialkylamino en C₁-C₁₂, un radical aryle ou un hétéroaryle éventuellement substitué, un substituant hydrosolubilisant choisi parmi un groupe carboxylate, un groupe sulfonate ou un reste ammonium ;
X désigne un atome d'hydrogène, un groupe COOR⁵ ou CONR⁶R⁷ ;
R⁵, R⁶ et R⁷, identiques ou différents, désignent un atome d'hydrogène, un radical alkyle en C1-C20, un radical alcènyle en C₂-C₁₀, un radical cycloalkyle en C₃-C₁₀, un radical cycloalcènyle en C₃-C₁₀, un groupe -(YO)o-Z ou un groupe aryle ;
Y désigne -(CH₂)₂-, -(CH₂)₃- -(CH₂)₄-, -CH-(CH₃)-CH₂- ;
Z représente-CH₂-CH₃, -CH₂CH₂CH₃, -CH₂-CH₂-CH₂-CH₃, -CH(CH₃)-CH₃ ;
m est un entier variant de 0 à 3 ;
n est un entier variant de 0 à 3 ;
o est un entier variant de 1 à 2 ;
pour la fabrication d'une composition cosmétique ou dermatologique, à usage topique, en particulier pour la photoprotection de la peau et des cheveux, qui comprend, dans un support cosmétiquement acceptable :
   (a) au moins un dérivé du dibénzoylméthane et
   (b) au moins un dérivé de 1,3,5-triazine photosensible en présence d'un dérivé du dibenzoylméthane répondant à la formule (I) suivante : 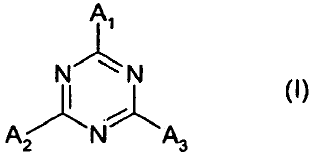
dans laquelle les radicaux A₁, A₂ et A₃, identiques ou différents sont choisis parmi les groupes de formules (II) : 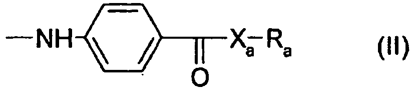 dans laquelle :
   - Xₐ, identiques ou différents, représentent l'oxygène ou le radical -NH-;
   - Rₐ, identiques ou différents, sont choisis parmi : l'hydrogène; un métal alcalin;
   un radical ammonium éventuellement substitué par un ou plusieurs radicaux alkyles ou hydroxyalkyles; un radical alkyle linéaire ou ramifié en C₁-C1₈; un radical cycloalkyle en C₅-C₁₂ éventuellement substitué par un ou plusieurs radicaux alkyles en C₁-C₄; un radical polyoxyéthyléné comprenant de 1 à 6 unités d'oxyde d'éthylène et dont le groupe OH terminal est méthyté ; un radical de formule (III), (IV) ou (V) suivantes : 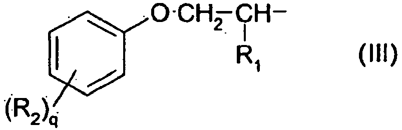 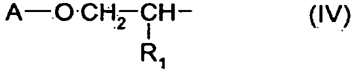 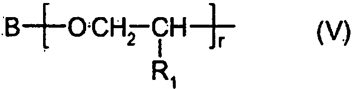
dans lesquelles :
   - R₁ est l'hydrogène ou un radical méthyle;
   - R₂ est un radical alkyle en C₁-C₉;
   - q est un nombre entier allant de 0 à 3;
   - r est un nombre entier allant de 1 à 10;
   - A est un radical alkyle en C₄-C₈ ou un radical cycloalkyle en C₅-C₈;
   - B est choisi parmi : un radical alkyle linéaire ou ramifié en C₁-C₈; un radical cycloalkyle en C₅-C₈; un radical aryle éventuellement substitué par un ou plusieurs radicaux alkyles en C₁-C₄ et
   (c) au moins ledit dérivé de 2-hydroxybenzophénone aminosubstitué; ladite composition ne contenant pas de p-méthylbenzytidènecamphre : le rapport en poids du dérivé de 2-hydroxybenzophénone sur le dérivé de dibenzoylméthane étant supérieur à 1, en vue d'améliorer dans ladite composition la stabilité au rayonnement UV dudit dérivé de 1,3,5-triazine.

Ainsi, selon la présente invention, on peut réaliser des compositions cosmétiques et/ou dermatologiques contenant du 4-tert-butyl-4'-méthoxydibenzoylméthane en association avec au moins un dérivé de 1,3,5-triazine photosensible, compositions dans lesquelles la concentration en dérivé de 1,3,5-triazine reste relativement constante même si ces compositions sont soumisses à l'action de la lumière.

Par quantité efficace de dérivé de 2-hydroxybenzophénone aminosubstitué conforme à l'invention, on entend une quantité suffisante pour obtenir une amélioration notable et significative de la photostabilité du dérivé de 1,3,5-triazine dans la composition cosmétique photoprotectrice. Cette quantité minimale en agent photostabilisant à mettre en oeuvre, qui peut varier selon la nature du support cosmétiquement acceptable retenu pour la composition, peut être déterminée sans aucune difficulté au moyen d'un test classique de mesure de photostabilité.

D'autres caractéristiques, aspects et avantages de la présente invention apparaîtront à la lecture de la description détaillée qui va suivre.

Le rapport en poids du dérivé de 2-hydroxybenzophénone sur le dérivé de dibenzoylméthane est supérieur à 1.

Un premier composé des compositions utilisables selon l'invention est donc un dérivé de 1,3,5-triazine photosensible en présence d'un dérivé du dibenzoylméthane.

Parmi les dérivés de 1,3,5-triazine utilisables dans le cadre de la présente invention, on utilise ceux répondant à la formule (I) suivante : 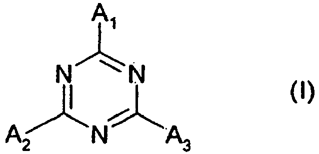 dans laquelle les radicaux A₁, A₂ et A₃, identiques ou différents sont choisis parmi les groupes de formules (II) : 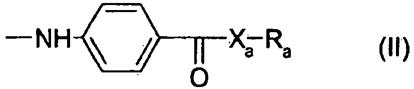 dans laquelle :
- Xₐ, identiques ou différents, représentent l'oxygène ou le radical -NH-;
- Rₐ, identiques ou différents, sont choisis parmi : l'hydrogène; un métal alcalin; un radical ammonium éventuellement substitué par un ou plusieurs radicaux alkyles ou hydroxyalkyles; un radical alkyle linéaire ou ramifié en C₁-C₁₈; un radical cycloalkyle en C₅-C₁₂ éventuellement substitué par un ou plusieurs radicaux alkyles en C₁-C₄; un radical polyoxyéthyléné comprenant de 1 à 6 unités d'oxyde d'éthylène et dont le groupe OH terminal est méthylé ; un radical de formule (III), (IV) ou (V) suivantes : 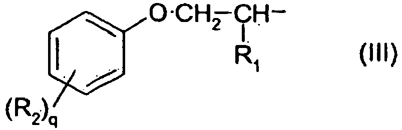 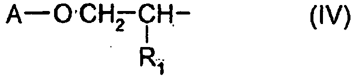 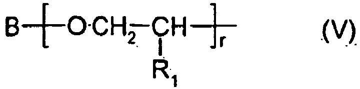 dans desquelles :
- R₁ est l'hydrogène ou un radical méthyle;
- R₂ est un radical alkyle en C₁-C₉;
- q est un nombre entier allant de 0 à 3;
- r est un nombre entier allant de 1 à 10;
- A est un radical alkyle en C₄-C₈ ou un radical cycloalkyle en C₅-C₈ ;
- B est choisi parmi : un radical alkyle linéaire ou ramifié en C₁-C₈; un radical cycloalkyle eh C₅-C₈; un radical aryle éventuellement substitué par un ou plusieurs radicaux alkyles en C₁-C₄.

Une première famille plus particulièrement préférée de dérivés de 1,3,5-triazine, notamment décrite dans le document EP-A-0 517 104, est celle des 1,3,5-triazines répondant à la formule (I) dans laquelle les A₁, A₂ et A₃ sont de formule (II) et présentent les caractéristiques suivantes :
- un Xₐ-Rₐ représente le radical -NH-Rₐ avec Rₐ choisi parmi : un radical cycloalkyle en C₅-C₁₂ éventuellement substitué par un ou plusieurs radicaux alkyles en C₁-C₄; un radical de formule (III), (IV) ou (V) ci-dessus dans lesquelles :
- B est un radical alkyle en C₁-C₄ ;
- R₂ est le radical méthyle ;
- les 2 autres Xₐ-Rₐ représentent le radical -O-Rₐ avec Rₐ, identiques ou différents choisis parmi : l'hydrogène; un métal alcalin; un radical ammonium éventuellement substitué par un ou plusieurs radicaux alkyles ou hydroxyalkyles; un radical alkyle linéaire ou ramifié en C₁-C₁₈; un radical cycloalkyle en C₅-C₁₂ éventuellement substitué par un ou plusieurs radicaux alkyles en C₁-C₄; un radical de formule (III), (IV) ou (V) ci-dessus dans lesquelles :
- B est un radical alkyle en C₁-C₄;
- R₂ est le radical méthyle.

Une deuxième famille plus particulièrement préférée de dérivés de 1,3,5-triazine, notamment décrite dans le document EP-A-0 570 838, est celle des 1,3,5-triazines répondant à la formule (I) dans laquelle les A₁, A₂ et A₃ sont de formule (II) et présentant l'ensemble des caractéristiques suivantes :
- un ou deux Xₐ-Rₐ représente le radical -NH-Rₐ, avec Rₐ choisi parmi : un radical alkyle linéaire ou ramifié en C₁-C₁₈; un radical cycloalkyle en C₅-C₁₂ éventuellement substitué avec un ou plusieurs radicaux alkyles en C₁-C₄; un radical de formule (III), (IV) ou (V) ci-dessus dans lesquelles :
- B est un radical alkyle en C₁-C₄ ;
- R₂ est le radical méthyle ;
le ou les deux autres Xₐ-Rₐ étant le radical -O-Rₐ avec Rₐ, identiques ou différents choisis parmi : l'hydrogène; un métal alcalin; un radical ammonium éventuellement substitué par un ou plusieurs radicaux alkyles ou hydroxyalkyles; un radical alkyle linéaire ou ramifié en C₁-C₁₈; un radical cycloalkyle en C₅-C₁₂ éventuellement substitué par un ou plusieurs radicaux alkyles en C₁-C₄; un radical de formule (III), (IV) ou (V) ci-dessus dans lesquelles :
- B est un radical alkyle en C₁-C₄;
- R₂ est le radical méthyle.

Une 1,3,5-triazine particulièrement préférée de cette deuxième famille est la 2-[(p-(tertiobutylamido)anilino]=4,6=bis-[(p-(2'=éthylhexyl=1'-oxycarbonyl)anilino]-1,3,5-triazine ou « Diethylhexyl Butamido Triazone» vendue sous le nom commercial «UVASORB HEB» par SIGMA 3V et répondant à la formule suivante : 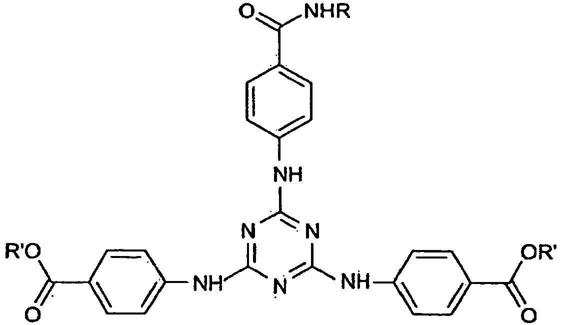 dans laquelle R' désigne un radical éthyl-2 hexyle et R désigne un radical tert-butyle.

Une troisième famille préférée de composés utilisables dans le cadre de la présente invention, et qui est notamment décrite dans le document US 4,724,137, est cette des 1,3,5-triazines répondant à la formule (I) dans laquelle les A₁, A₂ et A₃ sont de formule (II) et présentent les caractéristiques suivantes :
- Xₐ sont identiques et représentent l'oxygène;
- Rₐ, identiques ou différents, représentent un radical alkyle en C₆-C₁₂ ou un radical polyoxyéthyléné comprenant de 1 à 6 unités d'oxyde d'éthylène et dont le groupe OH terminal est méthylé.

Une 1,3,5-triazine particulièrement préférée de cette troisième famille est la 2,4;6-tris[p=(2'-éthylhexyl-1'-oxycarbonyl)anilino]-1,3,5-triazine ou «Ethylhexyl Triazone» vendue notamment sous la dénomination commerciale de "UVINUL T 150" par la Société BASF et répondant à la formule suivante : 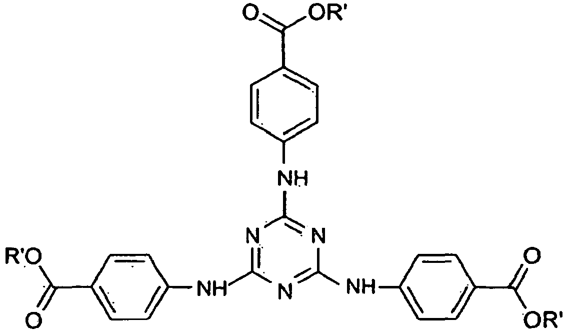 dans laquelle R' désigne un radical 2-éthyl hexyle.

Le ou les dérivés de 1,3,5-triazine sont généralement présents dans les compositions utilisables selon l'invention à une teneur pouvant aller de 0,5 % à 15 %, de préférence de 1 % à 10 % en poids, par rapport au poids total de la composition.

Un deuxième composé des compositions utilisable selon la présente invention est le dérivé de dibenzoylméthane. Comme indiqué précédemment, les dérivés du dibenzoylméthane visés par la présente invention sont des produits déjà bien connus en soi et décrits notamment dans les documents FR-A- 2 326 405, FR-A- 2 440 933 et EP-A-0 114 607, documents dont les enseignements sont, pour ce qui touche à la définition même de ces produits, totalement inclus à titre de références dans la présente description.

Parmi les dérivés du dibenzoylméthane plus particulièrement visés par la présente invention, on peut notamment citer, de manière non limitative :
- le 2-méthyldibenzoylméthane,
- le 4-méthyldibenzoylméthane,
- le 4-isopropyldibenzoylméthane,
- le 4-tert.-butyldibenzoylméthane,
- le 2,4-diméthyldibenzoylméthane,
- le 2,5-diméthyldibenzoylméthane,
- le 4,4'-diisopropyldibenzoylméthane,
- 4-tert-butyl-4'-méthoxydibenzoylméthane,
- le 2-méthyl-5-isopropyl-4'-méthoxydibenzoylméthane,
- le 2-méthyl-5-tert.-butyl-4'-méthoxydibenzoylméthane,
- le 2,4-diméthyl-4'-méthoxydibenzoylméthane,
- le 2,6-diméthyl-4-tert-butyl-4'-méthoxydibenzoylméthane.

Parmi les dérivés du dibenzoylméthane mentionnés ci-dessus, on préfère tout particulièrement, selon la présente invention, mettre en oeuvre le 4-tert-butyl=4'-méthoxydibenzoylméthane, notamment celui proposé à la vente sous la dénomination commerciale de "PARSOL 1789" par la Société Hoffmann Laroche, ce filtre répondant à la formule développée (VI) suivante : :

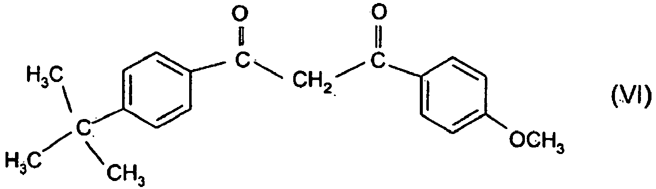

Un autre dérivé du dibenzoylméthane préféré selon la présente invention est le 4-isopropyldibenzoylméthane, filtre vendu sous la dénomination de "EUSOLEX 8020" par la Société Merck, et répondant à la formule développée (VII) suivante

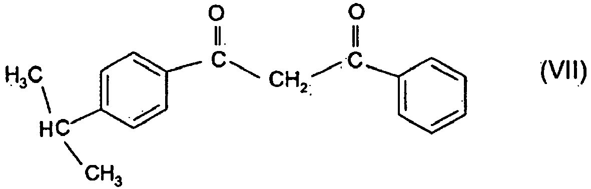

Le ou les dérivés du dibenzoylméthane sont présents dans les compositions utilisables conformément à l'invention à des teneurs de préférence allant de 0,5 à 15% en poids et plus préférentiellement de 1 % à 10 % en poids, par rapport au poids total de la composition.

Les dérivés de 2-hydroxybenzophénone aminosubstitués conformes à l'invention répondent à la formule (VIII) suivante : 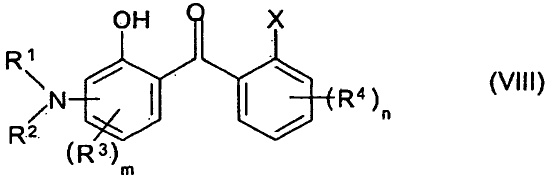 dans laquelle :
R¹ et R , identiques ou différents, désignent un atome d'hydrogène, un radical alkyle en C₁-C₂₀, un radical alcènyle en C₂-C₁₀, un radical cycloalkyle en C₃-C₁₀, un radical cycloalcènyle en C₃-C₁₀ ;
R¹ et R² peuvent également former avec l'atome d'azote avec lequel ils sont liés un cycle à 5 ou 6 chaînons ;
R³ et R⁴, identiques ou différents, désignent un radical alkyle en C₁-C₂₀, un radical alcènyle en C₂-C₁₀, un radical cycloalkyle en C₃-C₁₀, un radical cycloalcènyle en C₃-C₁₀, un radical alcoxy en C₁-C₁₂, un radical (C₁-C₂₀)alcoxycarbonyle, un radical alkylamino en C₁-C₁₂, un radical dialkylamino en C₁-C₁₂, un radical aryle ou un hétéroaryle éventuellement substitué, un substituant hydrosolubilisant choisi parmi un groupe carboxylate, un groupe sulfonate ou un reste ammonium ;
X désigne un atome d'hydrogène, un groupe COOR⁵ ou CONR⁶R⁷ ;
R⁵, R⁶ et R⁷, identiques ou différents, désignent un atome d'hydrogène, un radical alkyle en C1-C20, un radical alcènyle en C₂-C₁₀, un radical cycloalkyle en C₃-C₁₀, un radical cycloalcènyle en C₃-C₁₀, un groupe -(YO)o-Z ou un groupe aryle ;
Y désigne -(CH₂)₂-, -(CH₂)₃- -(CH₂)₄-, -CH-(CH₃)-CH₂- ;
Z représente-CH₂-CH₃, -CH₂CH₂CH₃, -CH₂-CH₂-CH₂-CH₃, -CH(CH₃)-CH₃ ;
m est un entier variant de 0 à 3 ;
n est un entier variant de 0 à 3 ;
o est un entier variant de 1 à 2.

Comme radicaux alkyle en C₁-C₂₀, on peut citer par exemple : méthyle, éthyle, n-propyle, 1-méthyléthyle, n-butyle, 1-méthylpropyle, 2-méthylpropyle, 1,1-diméthyléthyle, n-pentyte, 1-méthylbutyle, 2-méthylbutyle, 3-méthylbutyle, 2,2-diméthylpropyle, 1-éthylpropyle, n-hexyle, 1,1-diméthylpropyle, 1,2-diméthylpropyle, 1-méthylpentyle, 2-méthylpentyle, 3-méthylpentyle, 4-méthylpentyle, 1,1-diméthylbutyle, 1,2-diméthylbutyle, 1,3-diméthylbutyle, 2,2-diméthylbutyle, 2,3-diméthylbutyle, 3,3-diméthylbutyle, 1-éthylbutyle, 2-éthylbutyle, 1,2,2-triméthylpropyle, 1-éthyl-1-méthylpropyle, 1-éthyl-2-méthylpropyle, n-heptyle, n-octyle, n-nonyle, n-décyle, n-undécyle, n-dodécyle, n-tridécyle, n-tétradécyle, n-pentadécyle, n-hexadécyle, n-heptadécyle, n-octadécyle, n-nonadécyle ou n-eicosyle.

Comme groupes alcènyle en C₂-C₁₀, on peut citer par exemple vinyle, n-propènyle, isopropènyle, 1-butènyle, 2-butènyle, 1-pentènyle, 2-pentènyle, 2-méthyl-1-butènyle, .2-méthyl-2-butènyle, 3-méthyl-1-butènyle, 1-hexènyle, 2-hexènyle, 1-heptènyle, 2-heptènyle, 1-octènyle, 2-octènyle.

Comme radicaux alcoxy en C₁-C₁₂, on peut citer : méthoxy, éthoxy, n-propoxy, n-butoxy, n-pentoxy, 1-méthylpropoxy, 3-méthylbutoxy, 2,2-diméthylpropoxy, 1-méthyl- 1-éthylpropoxy, octoxy, 2-méthylpropoxy, 1,1-diméthylpropoxy, hexoxy, heptoxy, 2-éthylhexoxy.

Comme radicaux cycloalkyles en C₃-C₁₀, on peut citer par exemple : cyclopropyle, cyclobutyle, cyclopentyle, cyclohexyle, cycloheptyle, 1-méthylcyclopropyle, 1-éthylcyclopropyle, 1-propylcyclopropyle, 1-butylcyclopropyle, 1-pentylcyclopropyte, 1-méthyl-1-butylcyclopropyle, 1,2-diméthylcyclopropyle, 1-méthyl-2-éthylcyclopropyle, cyclooctyle, cyclononyle ou cyclodécyle.

Comme radicaux cycloalcènyles en C₃-C₁₀ ayant une ou plusieurs doubles liaisons, on peut citer : cyclopropènyle, cyclobutènyle, cyclopentènyle, cyclopentadiènyle, cyclohexènyle, 1,3-cyclohexadiènyle, 1,4-cyclohexadiènyle, cycloheptènyle, cycloheptatriènyle, cyclooctènyle, 1,5-cyclooctadiènyle, cyclooctatétraènyle, cyclononènyle ou cyclodécènyle.

Les radicaux cycloalkyles ou cycloalcényles peuvent comporter un ou plusieurs substituants (de préférence de 1 à 3) chois par exemple parmi halogène comme chlore, fluor ou brome ; cyano ; nitro ; amino ; C₁-C₄-alkylamino C₁-C₄ dialkylamino ; C₁-C₄alkyle ; C₁-C₄-alcoxy ; hydroxy ; ils peuvent également comporter de 1 à 3 hétéroatomes comme souffre, oxygène ou azote dont les valences libres puvent être occupées par un hydrogène ou un radical alkyle en C₁-C₄.

Les groupes aryles sont de préférence choisis parmi les cycles phényle ou naphtyle, lesquels peuvent comporter un ou plusieurs substituants (de préférence de 1 à 3) chois par exemple parmi halogène comme chlore, fluor ou brome ; cyano ; nitro ; amino ; C₁-C₄-alkylamino C₁-C₄ dialkylamino ; C₁-C₄alkyle ; C₁C₄-alcoxy ; hydroxy. On préfère plus particulièrement phényle, méthoxyphényle et naphtyle.

Les groupes hétéroaryles comportent en général un ou plusieurs hétéroatomes choisis parmi souffre, oxygène ou azote.

Les groupes hydrosolubilisants sont par exemple des groupes carboxylates, sulfonates et plus particulièrement leurs sels avec des cations physiologiquement acceptables comme les sels de métaux alcalins ou les sels de trialkylammonium comme les sels de tri(hydroxyalkyl)ammonium ou de 2-méthylpropan-1-ol-2-ammonium. On peut également citer les groupes ammonium comme les alkylammoniums et leurs formes salifiées avec des anions physiologiquement acceptables.

Comme exemples de cycle à 5 ou 6 chaînons formé par les radicaux R¹ et R² avec l'atome d'azote, on peut citer en particulier pyrrolidine ou pipéridine.

Les groupes amino peuvent être fixés sur le noyau benzénique en position ortho, méta ou para par rapport au radical carbonyle et plus préférentiellement en para.

Une famille de composés de formule (VIII) préférentiels comprend ceux choisis parmi ceux de formule (VIIIa) suivante : 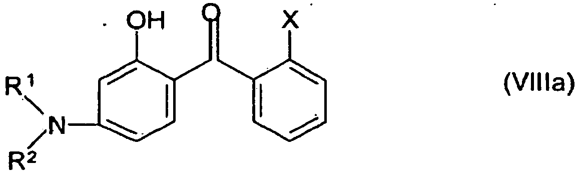 dans laquelle :
R¹ et R , identiques ou différents, désignent un atome d'hydrogène, un radical alkyle en C₁-C₁₂ ou forment avec l'atome d'azote avec lequel ils sont liés un cycle à 5 ou 6 chaînons ;
X désigne COOR⁵ ou CONR⁶R⁷ ;
R⁵ désigne un atome d'hydrogène, un radical alkyle en C₁-C₁₂, un radical cycloalkyle en C₃-C₆.
R⁶ et R⁷, identiques ou différents, désignent un atome d'hydrogène, un radical alkyle en C₁-C₁₂, un radical cycloalkyle en C₅-C₆.

Les composés de formule (VIIIa) plus particulièrement préférés sont ceux pour lesquels :
R¹ et R², identiques ou différents, désignent un radical alkyle en C₁-C₄ et plus particulèrement éthyle ;
R⁵ désigne un radical alkyle en C₃-C₈,
R⁶ et R , identiques ou différents, désignent un radical alkyle en C₁-C₈,

Une autre famille de composés de formule (VIII) préférentiels comprend ceux choisis parmi ceux de formule (Vlllb) suivante : 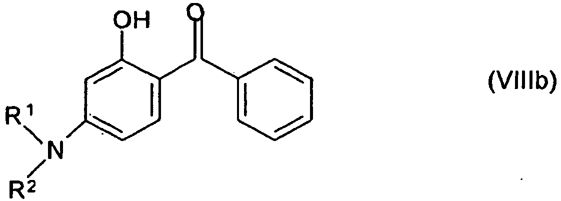 dans laquelle :
R¹ et R², identiques ou différents, désignent un radical alkyle en C₁-C₁₂ ou forment avec l'atome d'azote avec lequel ils sont liés un cycle à 5 ou 6 chaînons.

Parmi les composés de formule (Vlllb), on peut citer plus particulièrement :
- le (4-diéthylamino-2-hydroxyphényl)-phénylcétone.
- le (4-pyrrolidino-2-hydroxyphényl)-phénylcétone.

Une famille de composés de formule (VIII) plus particulièrement préférés comprend ceux choisis parmi ceux de formule (Vlllc) suivante : 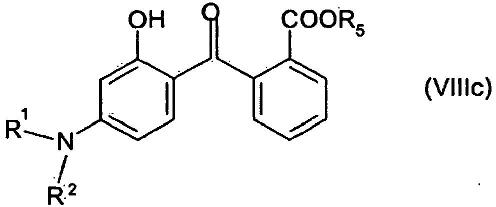 dans laquelle :
R¹ et R², identiques ou différents, désignent un atome d'hydrogène, un radical alkyle en C₁-C₈ ou forment avec l'atome d'azote avec lequel ils sont liés un cycle à 5 ou 6 chaînons ;
R⁵ désigne un atome d'hydrogène, un radical alkyle en C₁-C₁₂, un radical cycloalkyle en C₃-C₆.

Parmi les composés de formule (Vlllc), on peut citer :
- le 2-(4-pyrrolidino-2-hydroxybenzoyl)-benzoate
- le 2-(4-diéthylamino-2-hydroxybenzoyl)-benzoate de méthyle
- le 2-(4-diéthylamino-2-hydroxybenzoyl)-benzoate de 2-éthylhexyle
- le 2-(4-diéthylamino-2-hydroxybenzoyl)-benzoate de cyclohexyle
- le 2-(4-diéthylamino-2-hydroxybenzoyl)-benzoate de n-hexyle
- le 2-(4-dibutylamino-2-hydroxybenzoyl)-benzoate
- le 2-(4-dibutylamino-2-hydroxybenzoyl)-benzoate de méthyle
- le 2-(4-dibutylamino-2-hydroxybenzoyl)-benzoate d'isobutyle.

Un composé de formule (VIII) tout particulièrement préféré est le 2-(4-diéthylamino-2-hydroxybenzoyl)-benzoate de n-hexyle.

Les composés de formule (VIII) tels que définis ci-dessus sont connus en eux-mêmes et leurs structures et leurs synthèses sont décrites dans les demandes de brevet EP1046391-et DE100 12 408 (faisant partie intégrante du contenu de la description).

Les dérivés de 2-hydroxybenzophénone aminosubstitués conformes à l'invention sont présents de préférence dans la composition utilisable selon l'invention dans des proportions allant de 0,1 à 15 % en poids et plus préférentiellement de 1 à 10% en poids et plus particulièrement de 2 à 8% en poids par rapport au poids total de la composition.

Les compositions utilisables conformément à l'invention peuvent comporter en plus d'autres filtres UV organiques complémentaires actifs dans l'UVA et/ou l'UVB (absorbeurs), hydrosolubles ou liposolubles ou bien insolubles dans les solvants cosmétiques couramment utilisés.

Les filtres UV organiques complémentaires sont notamment choisis parmi les anthranilates ; les dérivés salicyliques ; les dérivés cinnamiques ; les dérivés du camphre autres que le p-méthylbenzylidènecamphre ; les dérivés de la benzophénone autres que ceux de formule (VIII) ; les dérivés de β,β'-diphénylacrylate, les dérivés de benzotriazole ; les dérivés de triazine non-photosensibles ; les dérivés de benzalmalonate ; les dérivés de benzimidazole ; les imidazolines ; les dérivés bis-benzoazolyle tels que décrits dans les brevets EP669323 et US 2,463,264; les dérivés de l'acide p-aminobenzoïque (PABA) ; les dérivés de méthylène bis-(hydroxyphényl benzotriazole) tels que décrits dans les demandes US5,237,071, US5,166,355, GB2303549, DE 197 26 184 et EP893119 ; les polymères filtres et silicones filtres tels que ceux décrits notamment dans la demande WO-93/04665 ; les dimères dérivés d'α-alkylstyrène tels que ceux décrits dans la demande de brevet DE19855649 ; les 4,4-diarylbutadiènes tels que décrits dans les demandes EP0967200, DE19746654, DE19755649, EP-A-1008586.

Comme exemples de filtres organiques complémentaires actifs dans l'UV-A et/ou l'UV-B, on peut citer désignés ci-dessus sous leur nom INCI :

### Dérivés de l'acide para-aminobenzoique :

- PABA,
- Ethyl PABA,
- Ethyl Dihydroxypropyl PABA,
- Ethylhexyl Diméthyl PABA vendu notamment sous le nom « ESCALOL 507 » par ISP,
- Glyceryl PABA,
- PEG-25 PABA vendu sous le nom « UVINUL P25 » par BASF,

### Dérivés salicyliques :

- Homosalate vendu sous le nom « EUSOLEX HMS » par RONA/EM INDUSTRIES,
- Ethylhexyl Salicylate vendu sous le nom « NEO HELIOPAN OS » par HAARMANN et REIMER,
- Dipropyleneglycol Salicylate vendu sous le nom « DIPSAL » par SCHER,
- TEA Salicylate, vendu sous le non « NEO HELIOPAN TS » par HAARMANN et REIMER.

### Dérivés cinnamiques :

- Ethylhexyl Methoxycinnamate vendu notamment sous le nom commercial « PARSOL MCX » par HOFFMANN LA ROCHE,
- Isopropyl Methoxy cinnamate,
- Isoamyl Methoxy cinnamate vendu sous le nom commercial « NEO HELIOPAN E 1000» par HAARMANN et REIMER,
- Cinoxate,
- DEA Methoxycinnamate,
- Diisopropyl Methylcinnamate,
- Glyceryl Ethylhexanoate Dimethoxycinnamate

### Dérivés de β,β-diphénylacrylate :

- Octocrylene vendu notamment sous le nom commercial « UVINUL N539 » par BASF,
- Etocrylene, vendu notamment sous le nom commercial « UVINUL N35 » par BASF,

### Dérivés de la benzophénone :

- Benzophenone-1 vendu sous le nom commercial « UVINUL 400 » par BASF,
- Benzophenone-2 vendu sous le nom commercial « UVINUL D50 » par BASF
- Benzophenone-3 ou Oxybenzone, vendu sous le nom commercial « UVINUL M40 » par BASF,
- Benzophenone-4 vendu sous le nom commercial « UVINUL MS40 » par BASF,
- Benzophenone-5
- Benzophenone-6 vendu sous le nom commercial « HELISORB 11 » par NORQUAY
- Benzophenone-8 vendu sous le nom commercial « SPECTRA-SORB UV-24 » PAR AMERICAN CYANAMID
- Benzophenone-9 vendu sous le nom commercial« UVINUL DS-49» par BASF,
- Benzophenone-12

### Dérivé du benzylidène camphre :

- 3-Benzylidene camphor fabriqué sous le nom « MEXORYL SD» par CHIMEX,
- Benzylidene Camphor Sulfonic Acid fabriqué sous le nom « MEXORYL SL» par CHIMEX,
- Camphor Benzalkonium Methosulfate fabriqué sous le nom « MEXORYL SO » par CHIMEX,
- Terephthalylidene Dicamphor Sulfonic Acid fabriqué sous le nom « MEXORYL SX » par CHIMEX,
- Polyacrylamidomethyl Benzylidene Camphor fabriqué sous le nom « MEXORYL SW » par CHIMEX,

### Dérivés de benzimidazole :

- Phenylbenzimidazole Sulfonic Acid vendu notamment sous le nom commercial « EUSOLEX 232 » par MERCK,
- Disodium Phenyl Dibenzimidazole Tetra-sulfonate vendu sous le nom commercial commercial « NEO HELIOPAN AP » par HAARMANN et REIMER,

### Dérivés de triazine :

- Anisotriazine vendu sous le nom commercial «TINOSORB S » par CIBA SPECIALTY CHEMICALS
- la 2,4,6-tris-(4'-amino benzalmalonate de diisobutyle)-s-triazine.

### Dérivés de benzotriazole :

- Drometrizole Trisiloxane vendu sous le nom « SILATRIZOLE » par RHODIA CHIMIE ,
- Methylène bis-Benzotriazolyl Tetramethylbutylphénol, vendu sous forme solide sous le nom commercial « MIXXIM BB/100 » par FAIRMOUNT CHEMICAL ou sous forme micronisé en dispersion aqueuse sous le nom commercial « TINOSORB M » par CIBA SPECIALTY CHEMICALS,

### Dérivés anthraniliques :

- Menthyl anthranilate vendu sous le nom commercial « NEO HELIOPAN MA » par HAARMANN et REIMER,

### Dérivés d'imidazolines :

- Ethylhexyl Dimethoxybenzylidene Dioxoimidazoline Propionate,

### Dérivés de benzalmalonate :

- Polyorganosiloxane à fonction benzalmalonate vendu sous la dénomination commerciale « PARSOL SLX » par HOFFMANN LA ROCHE et leurs mélanges.

### Dérivés de 4,4-diarylbutadiène :

- 1,1-dicarboxy (2,2'-diméthyl-propyl)-4,4-diphénylbutadiène et leurs mélanges.

Les filtres UV organiques plus particulièrement préférés sont choisis parmi les composés suivants :
- Ethylhexyl Salicylate,
- Ethylhexyl Methoxycinnamate
- Octocrylene,
- Phenylberizimidazole Sulfonic Acid,
- Benzophenone-3,
- Benzophenone-4,
- Benzophenone-5,
- 4-Methylbenzylidene camphor,
- Terephthalylidene Dicamphor Sulfonic Acid,
- Disodium Phenyl Dibenzimidazole Tetra-sutfonate,
- la 2,4,6-tris-(4'-amino benzalmalonate de diisobutyle)-s-triazine
- Anisotriazine,
- Methylène bis-Benzotriazolyl Tetramethylbutylphénol
- Drometrizole Trisiloxane
- 1,1-dicarboxy (2,2-diméthyl-proppyl)-4,4-diphénylbutadiène et leurs mélanges.

Les compositions cosmétiques utilisables selon l'invention peuvent encore contenir des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP-A-0518772 et EP-A-0518773.

Les compositions utilisables selon l'invention peuvent également contenir des agents de bronzage et/ou de brunissage artificiel de la peau (agents autobronzants), tels que par exemple de la dihydroxyacétone (DHA).

Les compositions utilisables selon l'invention peuvent comprendre en outre des adjuvants cosmétiques classiques notamment choisis parmi les corps gras, les solvants organiques, les épaississants ioniques ou non ioniques, les adoucissants, les antioxydants, les agents anti radicaux libres, les opacifiants, les stabilisants, les émollients, les silicones, les α-hydroxyacides, les agents anti-mousse, les agents hydratants, les vitamines, les agents répulsifs contre les insectes, les parfums, les conservateurs, les tensioactifs, les antiinflammatoires, les antagonistes de substance P, les charges, les polymères, les agents alcalinisants ou acidifiants, les colorants, les propulseurs ou tout autre ingrédient habituellement utilisé en cosmétique, en particulier pour la fabrication de compositions antisolaires sous forme d'émulsions.

Les corps gras peuvent être constitués par une huile ou une cire ou leurs mélanges. Par huile, on entend un composé liquide à température ambiante. Par cire, on entend un composé solide ou substantiellement solide à température ambiante, et dont le point de fusion est généralement supérieur à 35°C. Ils comprennent également les acides gras, les alcools gras et les esters d'acides gras, linéaires ou cycliques tels que les dérivés d'acide benzoïque, trimellitique et hydroxy-benzoïque.

Comme huiles, on peut citer les huiles minérales (paraffine); végétales (huile d'amande douce, de macadamia, de pépin de cassis, de jojoba) ; synthétiques comme le perhydrosqualène, les alcools, les acides ou les esters gras (comme le benzoate d'alcools en C₁₂-C₁₅ vendu sous la dénomination commerciale « Finsolv TN » par la société Finetex, le palmitate d'octyle, le lanolate d'isopropyle, les triglycérides dont ceux des acides caprique/capryliqué), les esters et éthers gras oxyéthylénés ou oxypropylénés; siliconées (cyclométhicone, polydiméthysiloxanes ou PDMS) ou fluorées, les polyalkylènes.

Comme composés cireux, on peut citer la paraffine, la cire de carnauba, la cire d'abeille, l'huile de ricin hydrogénée.

Parmi les solvants organiques, on peut citer les alcools et polyols inférieurs.

Bien entendu, l'homme de l'art veillera à choisir ce ou ces éventuels composés complémentaires et/ou leurs quantités de manière telle que les propriétés avantageuses, en particulier la photostabilité du dérivé de triazine, attachées intrinsèquement aux compositions utilisables conformément à l'invention ne soient pas, ou substantiellement pas, altérées par la ou les adjonctions envisagées.

Les compositions utilisables selon l'invention peuvent être préparées selon les techniques bien connues de l'homme de l'art, en particulier celles destinées à la préparation d'émulsions de type huile-dans-eau ou eau-dans-huile.

Ces compositions peuvent se présenter en particulier sous forme d'émulsion, simple ou complexe (H/E, E/H, H/E/H ou E/H/E) telle qu'une crème, un lait, un gel ou un gel crème, de poudre, de bâtonnet solide et éventuellement être conditionnée en aérosol et se présenter sous forme de mousse ou de spray.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR2315991 et FR2416008).

La composition cosmétique utilisable selon l'invention peut être utilisée comme composition protectrice de l'épiderme humain ou des cheveux contre les rayons ultraviolets, comme composition antisolaire ou comme produit de maquillage.

Lorsque la composition cosmétique utilisable selon l'invention est utilisée pour la protection de l'épiderme humain contre les rayons UV, ou comme composition antisolaires, elle peut se présenter sous forme de suspension ou de dispersion dans des solvants ou des corps gras, sous forme de dispersion vésiculaire non ionique ou encore sous forme d'émulsion, de préférence de type huile-dans-eau, telle qu'une crème ou un lait, sous forme de pommade, de gel, de gel crème, de bâtonnet solide, de poudre, de stick, de mousse aérosol ou de spray.

Lorsque la composition cosmétique utilisable selon l'invention est utilisée pour la protection des cheveux contre les rayons UV, elle peut se présenter sous forme de shampooing, de lotion, de gel, d'émulsion, de dispersion vésiculaire non ionique et constituer par exemple une composition à rincer, à appliquer avant ou après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage de coloration ou décoloration des cheveux.

Lorsque la composition est utilisée comme produit de maquillage des cils, des sourcils ou de la peau, tel que crème de traitement de l'épiderme, fond de teint, bâton de rouge à lèvres, fard à paupières, fard à joues, mascara ou ligneur encore appelé "eye liner", elle peut se présenter sous forme solide ou pâteuse, anhydre ou aqueuse, comme des émulsions huile dans eau ou eau dans huile, des dispersions vésiculaires non ioniques ou encore des suspensions.

A titre indicatif, pour les formulations antisolaires utilisables conformément à l'invention qui présentent un support de type émulsion huile-dans-eau, la phase aqueuse (comprenant notamment les filtrés hydrophiles) représente généralement de 50 à 95% en poids, de préférence de 70 à 90% en poids, par rapport à l'ensemble de la formulation, la phase huileuse (comprenant notamment les filtres lipophiles) de 5 à 50% en poids, de préférence de 10 à 30% en poids, par rapport à l'ensemble de la formulation, et le ou les (co)émulsionnant(s) de 0,5 à 20% en poids, de préférence de 2 à 10% en poids, par rapport à l'ensemble de la formulation.

Des exemples concrets illustrant l'invention, vont maintenant être donnés.

| **Exemple 1** | |
|---|---|
| Mélange d'alcool cétylstéarylique et d'alcool cétylstéarylique oxyéthyléné (33 OE) 80/20 (SINNOWAX AO -HENKEL) | 7g |
| Mélange de mono et distéarate de glycérol (CERASYNT SD-V ISP) | 2g |
| Alcool cétylique | 1.5g |
| Polydiméthyl siloxane (DOW CORNING 200 FLUID -DOW CORNING) | 1g |
| Benzoate d'alcools en C₁₂-C₁₅ (WITCONOL TN -WITCO) | 15g |
| 2-(4-diethylamino-2-hydroxybenzoyl)-benzoate de n-hexyle | 2g |
| Ethyl hexyl triazone (UVINUL T150 -BASF) | 2g |
| Butyl methoxydibenzoylmethane (PARSOL 1789 - HOFFMAN - LAROCHE) | 1.5g |
| Glycérine | 10g |
| Conservateurs | qs |
| Eau déminéralisée qsp | 100 g |

| **Exemple 2** | |
|---|---|
| Mélange mono /distéarate de glycerol / stéarate de polyéthylène glycol (100 OE) (ARLACEL 165 FL - ICI) | 2g |
| Alcool stéarylique (LANETTE 18 - HENKEL) | 1g |
| Acide stéarique d'huile de palme (STEARINE TP - STEARINERIE DUBOIS) | 2.5g |
| Poly diméthylsiloxane (DOW CORNING 200 FLUID - DOW CORNING) | 0.5g |
| Benzoate d'alcools en G12/C15 (WITCONOL TN -WITCO) | 15g |
| Triéthanolamine | 0.5g |
| 2-(4-diethylamino-2-hydroxybenzoyl)-benzoate de n-hexyle | 1.5g |
| Ethyl hexyl triazone (UVINUL T150 - BASF) | 2g |
| Butyl methoxydibenzoylmethane (PARSOL 1789 - HOFFMAN - LAROCHE) | 1g |
| Glycérine | 5g |
| Phosphate d'alcool hexadécylique, sel de potassium (AMPHISOL K - HOFFMAN LAROCHE) | 1g |
| Acide polyacrylique (SYNTHALEN K - 3V) | 0.3g |
| Hydroxypropyl méthyl cellulose (METHOCEL F4M -DOW CHEMICAL) | 0.1g |
| Triethanolamine | qs pH 7 |
| Conservateurs | qs |
| Eau déminéralisée qsp | 100 g |

## Revendications

1. Utilisation d'un dérivé de 2-hydroxybenzophénone aminosubstitué de formule (VIII) suivante 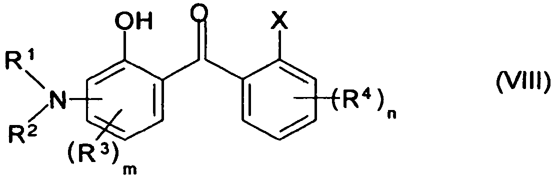 dans laquelle :
R¹ et R , identiques ou différents, désignent un atome d'hydrogène, un radical alkyle en C₁-C₂₀, un radical alcènyle en C₂-C₁₀, un radical cycloalkyle en C₃-C₁₀, un radical cycloalcènyle en C₃-C₁₀ ;
R¹ et R² peuvent également former avec l'atome d'azote avec lequel ils sont liés un cycle à 5 ou 6 chaînons;
R³ et R⁴, identiques ou différents, désignent un radical alkyle en C₁-C₂₀, un radical alcènyle en C₂-C₁₀, un radical cycloalkyle en C₃-C₁₀, un radical cycloalcènyle en C₃-C₁₀, un radical alcoxy en C₁-C₁₂, un radical (C₁-C₂₀)alcoxycarbonyle, un radical alkylamino en C₁-C₁₂, un radical dialkylamino en C₁-C₁₂, un radical aryle ou un hétéroaryle éventuellement substitué, un substituant hydrosolubilisant choisi parmi un groupe carboxylate,
un groupe sulfonate ou un reste ammonium ;
X désigne un atome d'hydrogène, un groupe COOR⁵ ou CONR⁶R⁷;
R⁵, R⁶ et R⁷, identiques ou différents, désignent un atome d'hydrogène, un radical alkyle en C1-C20, un radical alcènyle en C₂-C₁₀, un radical cycloalkyle en C₃-C₁₀, un radical cycloalcènyle en C₃-C₁₀, un groupe -(YO)o-Z ou un groupe aryle ;
Y désigne -(CH₂)₂-, -(CH₂)₃- -(CH₂)₄-, -CH-(CH₃)-CH₂- ;
Z représente -CH₂-CH₃, -CH₂CH₂CH₃-, -CH₂-CH₂-CH₂-CH₃, -CH(CH₃)-CH₃ ;
m est un entier variant de 0 à3 ;
n est un entier variant de 0 à 3 ;
o est un entier variant de 1 à 2 ;
pour la fabrication d'une composition cosmétique ou dermatologique, à usage topique, en particulier pour la photoprotection de la peau et des cheveux, qui comprend, dans un support cosmétiquement acceptable :
(a) au moins un dérivé du dibenzoylméthane et
(b) au moins un dérivé de 1,3,5-triazine photosensible en présence d'un dérivé du dibenzoylméthane répondant à la formule (I) suivante : 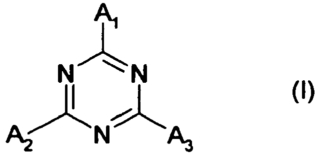
dans laquelle les radicaux A₁, A₂ et A₃, identiques ou différents sont choisis parmi les groupes de formules (II) : 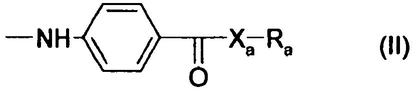
dans laquelle :
- Xₐ, identiques ou différents, représentent l'oxygène ou le radical -NH-;
- Rₐ, identiques ou différents, sont choisis parmi : l'hydrogène; un métal alcalin; un radical ammonium éventuellement substitué par un ou plusieurs radicaux alkyles ou hydroxyalkyles; un radical alkyle linéaire ou ramifié en C₁-C₁₈; un radical cycloalkyle en C₅-C₁₂ éventuellement substitué par un ou plusieurs radicaux alkyles en C₁-C₄; un radical polyoxyéthyléné comprenant de 1 à 6 unités d'oxyde d'éthylène et dont le groupe OH terminal est méthylé ; un radical de formule (III), (IV) ou (V) suivantes : 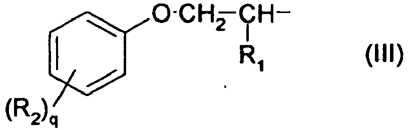 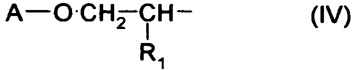 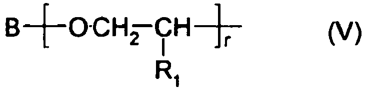
dans lesquelles :
- R₁ est l'hydrogène ou un radical méthyle;
- R₂ est un radical alkyle en C₁-C₉;
- q est un nombre entier allant de 0 à 3;
- r est un nombre entier allant de 1 à 10;
- A est un radical alkyle en C₄-C₈ ou un radical cycloalkyle en C₅-C₈ ;
- B est choisi parmi : un radical alkyle linéaire ou ramifié en C₁-C₈; un radical cycloalkyle en C₅-C₈; un radical aryle éventuellement substitué par un ou plusieurs radicaux alkyles en C₁-C₄ et
(c) au moins ledit dérivé de 2-hydroxybenzophénone aminosubstitué, ladite composition ne contenant pas de p-méthylbenzylidènecamphre ; le rapport en poids du dérivé de 2- hydroxybenzophénone sur le dérivé de dibenzoylméthane étant supérieur à 1,
en vue d'améliorer dans ladite composition la stabilité au rayonnement UV dudit dérivé de 1,3,5-triazine.

2. Utilisation selon la revendication 1, **caractérisée par le fait que** le dérivé de 1,3,5-triazine est choisi parmi ceux où les A₁, A₂ et A₃ sont de formule (II) et présentant l'ensemble des caractéristiques suivantes :
- un ou deux Xₐ-Rₐ représente le radical -NH-Rₐ, avec Rₐ choisi parmi : un radical alkyle linéaire ou ramifié en C₁-C₁₈; un radical cycloalkyle en C₅-C₁₂ éventuellement substitué avec un ou plusieurs radicaux alkyles en C₁-C₄; un radical de formule (III), (IV) ou (V) ci-dessus dans lesquelles :
- B est un radical alkyle en C₁-C₄;
- R₂ est le radical méthyle ;
le ou les deux autres Xₐ-Rₐ étant le radical -O-Rₐ avec Rₐ, identiques ou différents choisis parmi : l'hydrogène; un métal alcalin; un radical ammonium éventuellement substitué par un ou plusieurs radicaux alkyles ou hydroxyalkyles; un radical alkyle linéaire ou ramifié en C₁-C₁₈; un radical cycloalkyle en C₅-C₁₂ éventuellement substitué par un ou plusieurs radicaux alkyles en C₁-C₄; un radical de formule (III), (IV) ou (V) ci-dessus dans lesquelles :
- B est un radical alkyle en C₁-C₄;
- R₂ est le radical méthyle.

3. Utilisation selon la revendication 2, **caractérisée par le fait que** le dérivé de 1,3,5-triazine est celui répondant à la formule suivante : 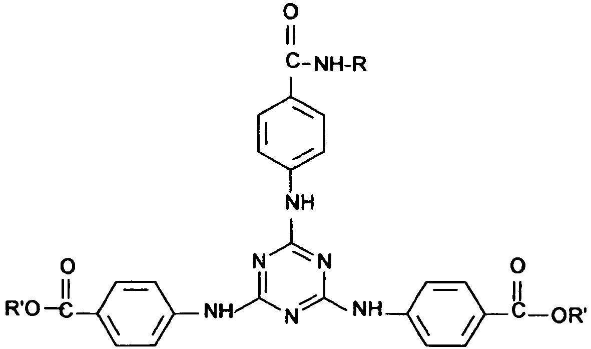 dans laquelle R' désigne un radical éthyl-2 hexyle et R désigne un radical tertio-butyle.

4. Utilisation selon la revendication 1, **caractérisée par le fait que** le dérivé de 1,3,5-triazine répondant à la formule (I) est choisi parmi ceux où les A₁, A₂ et A₃ sont de formule (II) et présentent les caractéristiques suivantes :
- Xₐ sont identiques et représentent l'oxygène;
- Rₐ, identiques ou différents et représentent un radical alkyle en C₆-C₁₂ ou un radical polyoxyéthyléné comprenant de 1 à 6 unités d'oxyde d'éthylène et dont le groupe OH terminal est méthylé.

5. Utilisation selon la revendication 4, **caractérisée par le fait que** le dérivé de 1,3,5-triazine est celui répondant à la formule suivante : 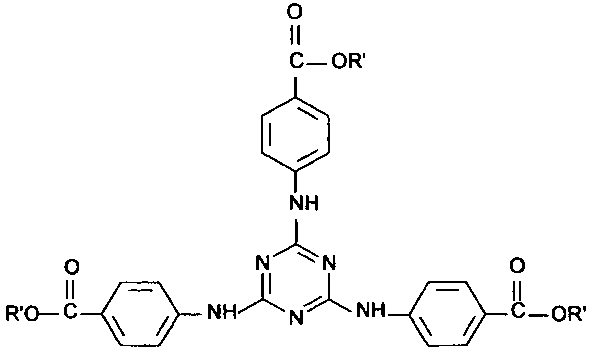 dans laquelle R' désigne un radical 2-éthyl hexyle.

6. Utilisation selon l'une quelconque des revendications 1 à 5, **caractérisée par le fait que** le dérivé de 1,3,5-triazine est présent dans la composition à des teneurs allant de 0,5 à 15% en poids et de préférence de 1 % à 10 % en poids, par rapport au poids total de la composition.

7. Utilisation selon l'une quelconque des revendications 1 à 6, **caractérisée par le fait que** le dérivé de dibenzoylméthane est choisi parmi :
- le 2-méthyldibenzoylméthane
- le 4-méthyldibenzoylméthane
- le 4-isopropyldibenzoylméthane
- le 4-tert.-butyldibenzoylméthane
- le 2,4-diméthyldibenzoylméthane
- le 2,5-diméthyldibenzoylméthane
- le 4,4'-düsopropyldibenzoylméthane
- le 4,4'-diméthoxydibenzoylméthane
- le 4-tert.-butyl-4'-méthoxydibenzoylméthane
- le 2-méthyl-5-isopropyl-4'-méthoxydibenzoylméthane
- le 2-méthyl-5-tert.-butyl-4'-méthoxydibenzoylméthane
- le 2,4-diméthyl-4'-méthoxydibenzoylméthane
- le 2,6-diméthyl-4-tert.-butyl-4'-méthoxydibenzoylméthane.

8. Utilisation selon la revendication 7, **caractérisée par le fait que** le dérivé de dibenzoylméthane est le 4-(ter.-butyl) 4'-méthoxy dibenzoylméthane.

9. Utilisation selon la revendication 7, **caractérisée par le fait que** le dérivé de dibenzoylméthane est le 4-isopropyl-dibenzoylméthane.

10. Utilisation selon l'une quelconque des revendications 1 à 9, où le dérivé du dibenzoylméthane est présent dans la composition à des teneurs allant de 0,5 à 15% en poids et de préférence de 1 % à 10 % en poids, par rapport au poids total de la composition.

11. Utilisation selon l'une quelconque des revendications 1 à 10 où le ou les composés de formule (VIII) sont choisis parmi ceux de formule (VIIIa) 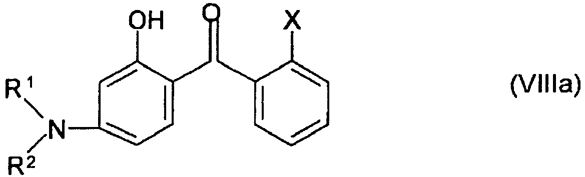 suivante :
dans laquelle :
R¹ et R , identiques ou différents, désignent un atome d'hydrogène, un radical alkyle en C₁-C₁₂ ou forment avec l'atome d'azote avec lequel ils sont liés un cycle à 5 ou 6 chaînons ;
X désigne COOR⁵ ou CONR⁶R⁷;
R⁵ désigne un atome d'hydrogène, un radical alkyle en C₁-C₁₂, un radical cycloalkyle en C₃-C₆.
R⁶ et R⁷, identiques ou différents, désignent un atome d'hydrogène, un radical alkyle en C₁-C₁₂, un radical cycloalkyle en C₅-C₆.

12. Utilisation selon la revendication 11, où les composés de formule (VIIIa) sont ceux pour lesquels :
R¹ et R², identiques ou différents, désignent un radical alkyle en C₁-C₄ et plus particulèrement éthyle ;
R⁵ désigne un radical alkyle en C₃-C₈,
R⁶ et R⁷ identiques ou différents, désignent un radical alkyle en C₁-C₈,

13. Utilisation selon selon l'une quelconque des revendications 1 à 10 où le ou les composés de formule (VIII) sont choisis parmi ceux de formule (VIIIb) suivante : 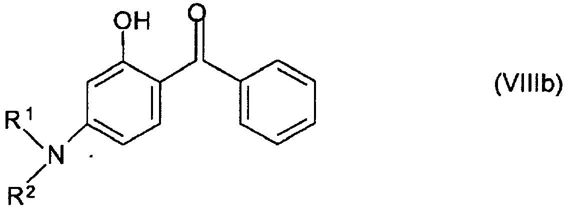 dans laquelle:
R¹ et R², identiques ou différents, désignent un radical alkyle en C₁-C₁₂ ou forment avec l'atome d'azote avec lequel ils sont liés un cycle à 5 ou 6 chaînons.

14. Utilisation selon la revendication 13, où le composé de formule (VIIIb) est choisi parmi :
- le (4-diéthylamino-2-hydroxyphényl)-phénylcétone.
- le (4-pyrrolidino-2-hydroxyphényl)-phénylcétone.

15. Utilisation selon l'une quelconque des revendications 1 à 10 où le ou les composés de formule (VIII) sont choisis parmi ceux de formule (VIIIc) suivante : 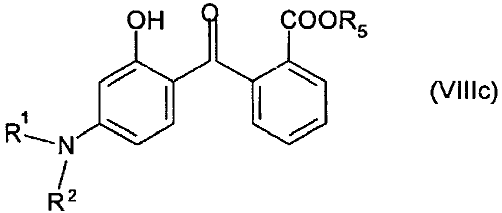 dans laquelle :
R¹ et R², identiques ou différents, désignent un atome d'hydrogène, un radical alkyle en C₁-C₈ ou forment avec l'atome d'azote avec lequel ils sont liés un cycle à 5 ou 6 chaînons ;
R⁵ désigne un atome d'hydrogène, un radical alkyle en C₁-C₁₂, un radical cycloalkyle en C₃-C₆.

16. Utilisation selon la revendication 15 où le composé de formule (VIIIc) est choisi parmi :
- le 2-(4-pyrrolidino-2-hydroxybenzoyl)-benzoate
- le 2-(4-diéthylamino-2-hydroxybenzoyl)-benzoate de méthyle
- le 2-(4-diéthylamino-2-hydroxybenzoyl)-benzoate de 2-éthylhexyle
- le 2-(4-diéthylamino-2-hydroxybenzoyl)-benzoate de cyclohexyle
- le 2-(4-diéthylamino-2-hydroxybenzoyl)-benzoate de n-hexyle
- le 2-(4-dibutylamino-2-hydroxybenzoyl)-benzoate
- le 2-(4-dibutylamino-2-hydroxybenzoyl)-benzoate de méthyle
- le 2-(4-dibutylamino-2-hydroxybenzoyl)-benzoate d'isobutyle.

17. Utilisation selon la revendication 16 où le composé de formule (VIIIc) est le 2-(4-diéthylamino-2-hydroxybenzoyl)-benzoate de n-hexyle

18. Utilisation selon l'une quelconque des revendications 1 à 17, dans laquelle le ou les dérivés de 2-hydroxybenzophénone aminosubstitués de formule (VIII) sont présents dans des proportions allant de 0,1 % à 15 % en poids, par rapport au poids total de la composition.

19. Utilisation selon la revendication 18, dans laquelle le ou les dérivés de 2-hydroxybenzophénone aminosubstitués de formule (VIII) sont présents dans la composition dans des proportions allant de 1 à 10% en poids, par rapport au poids total de la composition.

20. Utilisation selon la revendication 19, dans laquelle le ou les dérivés de 2-hydroxybenzophénone aminosubstitués de formule (VIII) sont présents dans la composition dans des proportions allant de 2 à 8% en poids par rapport au poids total de la composition.

21. Utilisation selon l'une quelconque des revendications 1 à 20, **caractérisée par le fait que** la composition contient en plus d'autres filtres organiques actifs dans l'UV-A et/ou l'UV-B.

22. Utilisation selon la revendication 21, où le ou les filtres UV organiques complémentaires sont choisis parmi les anthranilates ; les dérivés salicyliques ; les dérivés cinnamiques ; les dérivés du camphre autres que le p-méthylbenzylidènecamphre ; les dérivés de la benzophénone autres que ceux de formule (VIII); les dérivés de β, β -diphénylacrylate, les dérivés de benzotriazole ; les dérivés de triazine non-photosensibles ; les dérivés de benzalmalonate ; les dérivés de benzimidazole ; les imidazolines ; les dérivés bis-benzoazolyle ; les dérivés de l'acide p-aminobenzoïque (PABA) ; les dérivés de méthylène bis-(hydroxyphényl benzotriazole) ; les polymères filtres et silicones ; les dimères dérivés d'α-alkylstyrène; les 4,4-diarylbutadiènes et leurs mélanges.

23. Utilisation selon la revendication 22, **caractérisée par le fait que** le ou les filtres UV organiques sont choisis parmi les composés suivants :
- Ethylhexyl Salicylate,
- Ethylhexyl Methoxycinnamate
- Octocrylene,
- Phenylbenzimidazole Sulfonic Acid,
- Benzophenone-3,
- Benzophenone-4,
- Benzophenone-5,
- 4-Methylbenzylidene camphor,
- Terephthalylidene Dicamphor Sulfonic Acid,
- Disodium Phenyl Dibenzimidazole Tetra-sulfonate,
- la 2,4,6-tris-(4'-amino benzalmalonate de diisobutyle)-s-triazine
- Anisotriazine,
- Methylène bis-Benzotriazolyl Tetramethylbutylphénol
- Drometrizole Trisiloxane
- -1,1-dicarboxy (2,2'-diméthyl-propyl)-4,4-diphénylbutadiène et leurs mélanges.

24. Utilisation selon l'une quelconque des revendications 1 à 20, **caractérisée par le fait que** la composition comprend en outre, des pigments ou des nanopigments d'oxydes métalliques, enrobés ou non.

25. Utilisation selon la revendication 24, **caractérisée par le fait que** lesdits pigments ou nanopigments sont choisis parmi les oxydes de titane, de zinc, de fer, de zirconium, de cérium et leurs mélanges, enrobés ou non.

26. Utilisation selon l'une quelconque des revendications 1 à 25, **caractérisée par le fait que** la composition comprend en outre au moins un agent de bronzage et/ou de brunissage artificiel de la peau.

27. Utilisation selon l'une quelconque des revendications 1 à 26, **caractérisée par le fait que** la composition comprend en outre au moins un adjuvant choisi parmi les corps gras, les solvants organiques, les épaississants ioniques ou non ioniques, les adoucissants, les antioxydants, les agents anti radicaux libres, les opacifiants, les stabilisants, les émollients, les silicones, les α-hydroxyacides, les agents anti-mousse, les agents hydratants, les vitamines, les agents répulsifs contre les insectes, les parfums, les conservateurs, les tensioactifs, les antiinflammatoires, les antagonistes de substance P, les charges, les polymères, les propulseurs, les agents alcalinisants ou acidifiants, les colorants.

28. Utilisation selon l'une quelconque des revendications 1 à 27, **caractérisée par le fait que** la composition est une composition protectrice de l'épiderme humain ou d'une composition antisolaire et qu'elle se présente sous forme d'une dispersion vésiculaire non ionique, d'une émulsion, en particulier d'une émulsion de type huile-dans-eau, d'une crème, d'un lait, d'un gel, d'un gel crème, d'une suspension, d'une dispersion, d'une poudre, d'un bâtonnet solide, d'une mousse ou d'un spray.

29. Utilisation selon l'une quelconque des revendications 1 à 27, **caractérisée par le fait que** la composition est une composition de maquillage des cils, des sourcils ou de la peau et qu'elle se présente sous forme solide ou pâteuse, anhydre ou aqueuse, d'une émulsion, d'une suspension ou d'une dispersion.

30. Utilisation selon l'une quelconque des revendications 1 à 27, **caractérisée par le fait que** la composition est une composition destinée à la protection des cheveux contre les rayons ultraviolets et qu'elle se présente sous la forme d'un shampooing, d'une lotion, d'un gel, d'une émulsion, d'une dispersion vésiculaire non ionique.

## Patentansprüche

1. Verwendung eines aminosubstituierten 2-Hydrozybenzophenon-Derivats der folgenden Formel (VIII) : 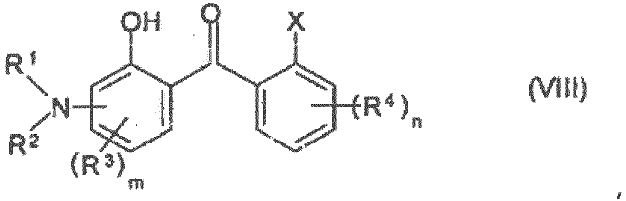 worin:
R¹ und R², die gleich oder verschieden sind, für ein Wasserstoffatom, einen C₁-C₂₀-Alkylrest, einen C₂-C₁₀-Alkenylrest, einen C₃-C₁₀-Cycloalkylrest, einen C₃-C₁₀-Cycloalkenylrest stehen;
R¹ und R² auch zusammen mit dem Stickstoffatom, mit dem sie verbunden sind, einen 5- oder 6-gliedrigen Ring bilden können;
R³ und R⁴, die gleich oder verschieden sind, für einen C₁-C₂₀-Alkylrest, einen C₂-C₁₀-Alkenylrest, einen C₃-C₁₀-Cycloalkylrest, einen C₃-C₁₀₋Cycloalkenylrest, einen C₁-C₁₂-Alkoxyrest, einen (C₁-C₂₀) Alkoxycarbonylrest, einen C₁-C₁₂-Alkylaminorest, einen C₁-C₁₂-Dialkylaminorest, einen Arylrest oder einen Heteroarylrest, der gegebenenfalls substituiert ist, einen wasserlöslich machenden Substituenten, der aus einer Carboxylatgruppe, einer Sulfonatgruppe oder einem Ammoniumrest ausgewählt ist, steht;
X für ein Wasserstoffatom, eine Gruppe -COOR⁵ oder -CONR⁶R⁷ steht;
R⁵, R⁶ und R⁷, die gleich oder verschieden sind, für ein Wasserstoffatom, einen C₁-C₂₀-Alkylrest, einen C₂-C₁₀-Alkenylrest, einen C₃-C₁₀-Cycloalkylrest, einen C₃-C₁₀-Cycloalkenylrest, eine Gruppe -(YO)o-Z oder eine Arylgruppe stehen;
Y für - (CH₂)₂ -, - (CH₂)₃-, -(CH₂)₄-, -CH-(CH₃)-CH₂- steht;
Z für -CH₂-CH₃, - CH₂CH₂CH₃, - -CH₂-CH₂-CH₂-CH₃, -CH(CH₃)-CH₃ steht;
m eine ganze Zahl von 0 bis 3 ist;
n eine ganze Zahl von 0 bis 3 ist;
o eine ganze Zahl von 1 bis 2 ist, zur Herstellung einer kosmetischen oder dermatologischen Zusammensetzung für die topische Verwendung, insbesondere zur Photoprotektion der Haut und der Haare, die in einem kosmetisch akzeptablen Träger Folgendes umfasst:
(a) mindestens ein Derivat von Dibenzoylmethan und
(b) mindestens ein lichtempfindliches 1,3,5-Triazin-Derivat in Gegenwart eines Derivats von Dibenzoylmethan mit der folgenden Formel (I): 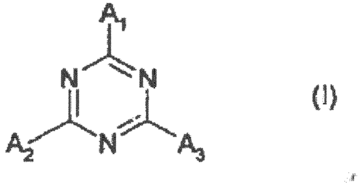
worin die Reste A₁, A₂ und A₃, die gleich oder verschieden sind, aus den Gruppen der Formeln (II) ausgewählt sind: 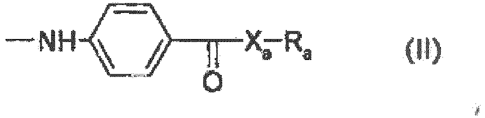
worin:
- Xₐ, die gleich oder verschieden sind, für Sauerstoff oder den Rest -NH- stehen;
- Rₐ, die gleich oder verschieden sind, aus Folgenden ausgewählt sind: Wasserstoff; ein Alkalimetall; einem Ammoniumrest, der gegebenenfalls mit einem oder mehreren Alkyl- oder Hydroxyalkylresten substituiert ist; einem geraden oder verzweigten C₁-C₁₈-Alkylrest; einem C₅-C₁₂-Cycloalkylrest, der gegebenenfalls mit einem oder mehreren C₁-C₄-Alkylresten substituiert ist; einem Polyoxyethylenrest, der 1 bis 6 Ethylenoxideinheiten umfasst und dessen endständige OH-Gruppe methyliert ist: und einen Rest der folgenden Formeln (III), (IV) oder (V): 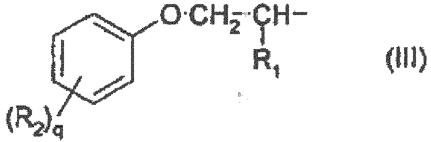 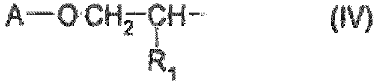 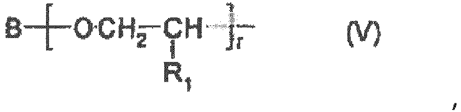
worin:
- R₁ Wasserstoff oder ein Methylrest ist;
- R₂ ein C₁-C₉-Alkylrest ist;
- q eine ganze Zahl von 0 bis 3 ist;
- r eine ganze Zahl von 1 bis 10 ist;
- A ein C₄-C₈-Alkylrest oder ein C₅-C₈-Cycloalkylrest ist;
- B aus Folgenden ausgewählt ist: einem geraden oder verzweigten C₁-C₈-Alkylrest; einem C₅-C₈-Cycloalkylrest; einem Arylrest, der gegebenenfalls mit einem oder mehreren C₁-C₄-Alkylresten substituiert ist, und (c) mindestens das aminosubstituierte 2-Hydroxybenzophenon-Derivat,
wobei die Zusammensetzung keinen p-Methylbenzylidenkampfer enthält, wobei das Verhältnis, bezogen auf Gewicht, des 2-Hydroxybenzophenon-Derivats zum Dibenzoylmethan-Derivat größer als 1, ist,
um in der Zusammensetzung die Beständigkeit des 1,3,5-Triazin-Derivats gegenüber UV-Strahlung zu verbessern.

2. Verwendung nach Anspruch 1, **dadurch gekennzeichnet, dass** das 1,3,5-Triazin-Derivat aus denjenigen ausgewählt ist, worin die Reste A₁, A₂ und A₃ die Formel (II) haben und die die Gesamtheit der folgenden Eigenschaften aufweisend:
- ein oder zwei Reste Xₐ-Rₐ stehen für den Rest -NH-Rₐ, wobei Rₐ aus Folgenden ausgewählt ist: einem geraden oder verzweigten C₁-C₁₈-Alkylrest; einem C₅-C₁₂-Cycloalkylrest, der gegebenenfalls mit einem oder mehreren C₁-C₄-Alkylresten substituiert ist; einem Rest der vorstehenden Formel (III), (IV) oder (V), worin:
- B ein C₁-C₄-Alkylrest ist;
- R₂ ein Methylrest ist;
- der oder die beiden anderen Reste Xₐ-Rₐ der Rest -O-Rₐ sind, worin Rₐ, die gleich oder verschieden sind, aus Folgenden ausgewählt sind: Wasserstoff; einem Alkalimetall; einem Ammoniumrest, der gegebenenfalls mit einem oder mehreren Alkyl- oder Hydroxyalkylresten substituiert ist; einem geraden oder verzweigten C₁-C₁₈-Alkylrest; einem C₅-C₁₂-Cycloalkylrest, der gegebenenfalls mit einem oder mehreren C₁-C₄-Alkylresten substituiert ist; einem Rest der vorstehenden Formel (III), (IV) oder (V), worin:
- B ein C₁-C₄-Alkylrest ist,
- R₂ ein Methylrest ist.

3. Verwendung nach Anspruch 2, **dadurch gekennzeichnet, dass** das 1,3,5-Triazin-Derivat dasjenige der folgenden Formel ist: 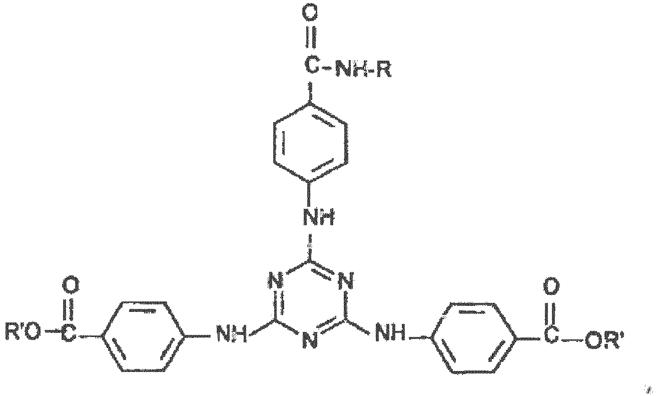 worin R' für einen 2-Ethylhexylrest steht und R für einen tert-Butylrest steht.

4. Verwendung nach Anspruch 1, **dadurch gekennzeichnet, dass** das 1,3,5-Triazin-Derivat die Formel (I) hat und aus denjenigen ausgewählt ist, worin die Reste A₁, A₂ und A₃ die Formel (II) haben und die folgenden Eigenschaften aufweisen:
- die Reste Xₐ sind gleich und stehen für Sauerstoff;
- die Reste Rₐ, gleich oder verschieden und stehen für einen C₆-C₁₂-Alkylrest oder einen Polyethoxylenrest, der 1 bis 6 Ethylenoxideinheiten umfasst und dessen endständige OH-Gruppe methyliert ist.

5. Verwendung nach Anspruch 4, **dadurch gekennzeichnet, dass** das 1,3,5-Triazin-Derivat dasjenige der folgenden Formel ist: 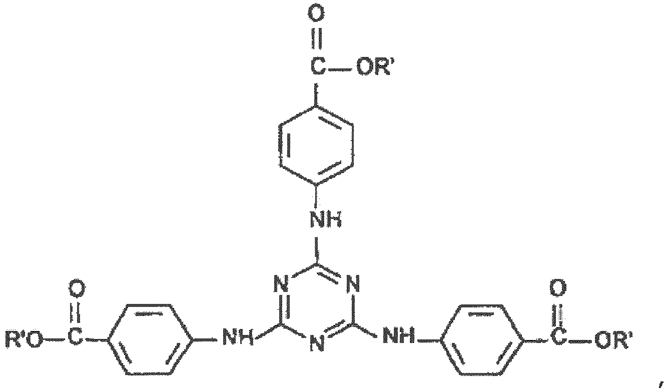 worin R' für einen 2-Ethylhexylrest steht.

6. Verwendung nach einem der Ansprüche 1 bis 5, **dadurch gekennzeichnet, dass** das 1,3,5-Triazin-Derivat in der Zusammensetzung in Konzentrationen von 0,5 bis 15 Gew.-% und vorzugsweise von 1 bis 10 Gew.-%, bezogen auf das Gesamtgewicht der Zusammensetzung, vorliegt.

7. Verwendung nach einem der Ansprüche 1 bis 6, **dadurch gekennzeichnet, dass** das Dibenzoylmethan-Derivat aus Folgenden ausgewählt ist:
- 2-Methyldibenzoylmethan
- 4-Methyldibenzoylmethan
- 4-Isopropyldibenzoylmethan
- 4-tert-Butyldibenzoylmethan
- 2,4-Dimethyldibenzoylmethan
- 2,5-Dimethyldibenzoylmethan
- 4,4'-Diisopropyldibenzoylmethan
- 4,4'-Dimethoxydibenzoylmethan
- 4-tert-Butyl-4'-methoxydibenzoylmethan
- 2-Methyl-5-isopropyl-4'-methoxydibenzoylmethan
- 2-Methyl-5-tert-butyl-4'-methoxydibenzoylmethan
- 2,4-Dimethyl-4'-methoxydibenzoylmethan
- 2,6-Dimethyl-4-tert-butyl-4'-methoxydibenzoylmethan.

8. Verwendung nach Anspruch 7, **dadurch gekennzeichnet, dass** es sich bei dem Dibenzoylmethan-Derivat um 4-(tert-Butyl)-4'-methoxydibenzoylmethan handelt.

9. Verwendung nach Anspruch 7, **dadurch gekennzeichnet, dass** es sich bei dem Dibenzoylmethan-Derivat um 4-Isopropyldibenzoylmethan handelt.

10. Verwendung nach einem der Ansprüche 1 bis 9, wobei das Derivat von Dibenzoylmethan in der Zusammensetzung in Konzentrationen von 0,5 bis 15 Gew.-% und vorzugsweise von 1 bis 10 Gew.-%, bezogen auf das Gesamtgewicht der Zusammensetzung, vorliegt.

11. Verwendung nach einem der Ansprüche 1 bis 10, wobei die Verbindung oder die Verbindungen der Formel (VIII) aus denjenigen der folgenden Formel (VIIIa) ausgewählt sind: 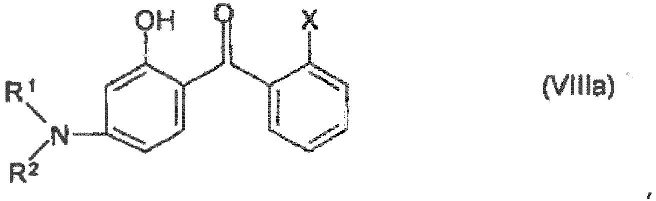 worin:
R¹ und R², die gleich oder verschieden sind, für ein Wasserstoffatom, einen C₁-C₁₂-Alkylrest stehen oder
zusammen mit dem Stickstoffatom, mit dem sie verbunden sind, einen 5- oder 6-gliedrigen Ring bilden;
X für COOR⁵ oder CONR⁶R⁷ steht;
R⁵ für ein Wasserstoffatom, einen C₁-C₁₂-Alkylrest, einen C₃-C₆-Cycloalkylrest steht,
R⁶ und R⁷, die gleich oder verschieden sind, für ein Wasserstoffatom, einen C₁-C₁₂-Alkylrest , einen C₅-C₆-Cycloalkylrest stehen.

12. Verwendung nach Anspruch 11, wobei die Verbindungen der Formel (VIIIa) diejenigen sind, bei denen
R¹ und R², die gleich oder verschieden sind, für einen C₁-C₄-Alkyl- und stärker bevorzugt Ethylrest stehen;
R⁵ für einen C₃-C₈-Alkylrest steht,
R⁶ und R⁷, die gleich oder verschieden sind, für einen C₁-C₈-Alkylrest stehen.

13. Verwendung nach einem der Ansprüche 1 bis 10, wobei die Verbindung oder die Verbindungen der Formel (VIII) aus denjenigen der folgenden Formel (VIIIb) ausgewählt sind: 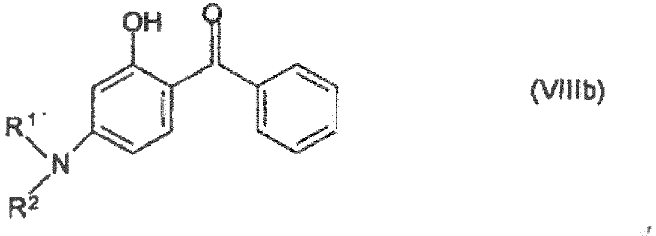 worin:
R¹ und R², die gleich oder verschieden sind, für einen C₁-C₁₂-Alkylrest stehen oder zusammen mit dem Stickstoffatom, mit dem sie verbunden sind, einen 5-oder 6-gliedrigen Ring bilden.

14. Verwendung nach Anspruch 13, wobei die Verbindung der Formel (VIIIb) aus Folgenden ausgewählt ist:
- (4-Diethylamino-2-hydroxyphenyl)-phenylketon
- (4-Pyrrolidino-2-hydroxyphenyl)-phenylketon.

15. Verwendung nach einem der Ansprüche 1 bis 10, wobei die Verbindung oder die Verbindungen der Formel (VIII) aus denjenigen der folgenden Formel (VIIIc) ausgewählt sind: 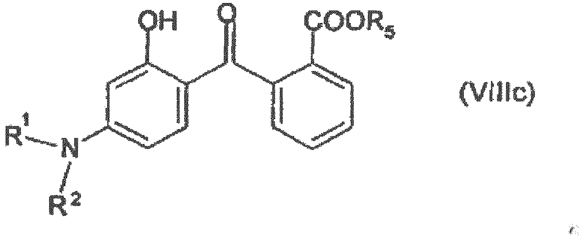 worin:
R¹ und R², die gleich oder verschieden sind, für ein Wasserstoffatom, einen C₁-C₈-Alkylrest stehen oder zusammen mit dem Stickstoffatom, mit dem sie verbunden sind, einen 5- oder 6-gliedrigen Ring bilden;
R⁵ für ein Wasserstoffatom, einen C₁-C₁₂-Alkylrest, einen C₃-C₆--Cycloalkylrest steht.

16. Verwendung nach Anspruch 15, wobei die Verbindung der Formel (VIIIc) aus Folgenden ausgewählt ist:
- 2-(4-Pyrrolidino--2-hydroxybenzoyl)-benzoat
- Methyl-2-(4-diethylamino-2-hydroxybenzoyl)-benzoat
- 2-Ethylhexyl-2-(4-diethylamino-2-hydroxybenzoyl)-benzoat
- Cyclohexyl-2-(4-diethylamino-2-hydroxybenzoyl)-benzoat
- n-Hexyl-2-(4-diethylamino-2-hydroxybenzoyl)-benzoat 2-(4-Dibutylamino-2-hydroxybenzoyl)-benzoat
- Methyl-2-(4-dibutylamino-2-hydroxybenzoyl)-benzoat
- Isobutyl-2-(4-dibutylamino-2-hydroxybenzoyl)-benzoat.

17. Verwendung nach Anspruch 16, wobei die Verbindung der Formel (VIIIc) n-Hexyl-2-(4-diethylamino-2-hydroxybenzoyl)-benzoat ist.

18. Verwendung nach einem der Ansprüche 1 bis 17, wobei das oder die aminosubstituierte(n) 2-Hydroxybenzophenon-Derivat(e) der Formel (VIII) in Anteilen von 0,1 bis 15 Gew.-%, bezogen auf das Gesamtgewicht der Zusammensetzung, vorliegen.

19. Verwendung nach Anspruch 18, wobei das oder die aminosubstituierte(n) 2-Hydroxybenzophenon-Derivat(e) der Formel (VIII) in der Zusammensetzung in Anteilen von 1 bis 10 Gew.-%, bezogen auf das Gesamtgewicht der Zusammensetzung, vorliegen.

20. Verwendung nach Anspruch 19, wobei das oder die aminosubstituierte(n) 2-Hydroxybenzophenon-Derivat(e) der Formel (VIII) in der Zusammensetzung in Anteilen von 2 bis 8 Gew.-%, bezogen auf das Gesamtgewicht der Zusammensetzung, vorliegen.

21. Verwendung nach einem der Ansprüche 1 bis 20, **dadurch gekennzeichnet, dass** die Zusammensetzung außerdem andere organische Filter enthält, die im UV-A- und/oder UV-B-Bereich aktiv sind.

22. Verwendung nach Anspruch 21, wobei der oder die zusätzliche(n) organische(n) UV-Filter aus Anthranilaten; Salicylsäure-Derivaten; Zimtsäure-Derivaten; anderen Kampfer-Derivaten als p-Methylbenzylidenkampfer; anderen Benzophenon-Derivaten als denjenigen der Formel (VIII); β,β-Diphenylacrylat-Derivaten; Benzotriazol-Derivaten; nicht lichtempfindlichen Triazin-Derivaten; Benzalmalonat-Derivaten; Benzimidazol-Derivaten; Imidazolinen; Bisbenzoazolyl-Derivaten; p-Aminobenzoesäure-(PABA)-Derivaten; Methylenbis(hydroxyphenylbenzotriazol)-Derivaten; Filterpolymeren und Silikonen; von α-Alkylstyrol herrührenden Dimeren; 4,4-Diarylbutadienen und deren Gemischen ausgewählt ist.

23. Verwendung nach Anspruch 22, **dadurch gekennzeichnet, dass** der oder die organische(n) UV-Filter aus den folgenden Verbindungen ausgewählt sind:
- Ethylhexylsalicylat,
- Ethylhexylmethoxycinnamat,
- Octocrylen,
- Phenylbenzimidazolsulfonsäure,
- Benzophenon-3,
- Benzophenon-4,
- Benzophenon-5,
- 4-Methylbenzylidenkampfer,
- Terephthalylidendikampfersulfonsäure,
- Dinatriumphenyldibenzimidazoltetrasulfonat,
- 2,4,6-Tris-(diisobutyl-4'-aminobenzalmalonat)-s-triazin,
- Anisotriazin,
- Methylenbisbenzotriazolyltetramethylbutylphenol,
- Drometrizoltrisiloxan,
- 1,1-Dicarboxy(2,2'-dimethylpropyl)-4,4-diphenylbutadien
und deren Gemischen.

24. Verwendung nach einem der Ansprüche 1 bis 20, **dadurch gekennzeichnet, dass** die Zusammensetzung außerdem überzogene oder nicht überzogene Metalloxidpigmente oder -nanopigmente umfasst.

25. Verwendung nach Anspruch 24, **dadurch gekennzeichnet, dass** die Pigmente oder Nanopigmente aus überzogenen oder nicht überzogenen Titan-, Zink-, Eisen-, Zirkon- oder Ceroxiden und deren Gemischen ausgewählt sind.

26. Verwendung nach einem der Ansprüche 1 bis 25, **dadurch gekennzeichnet, dass** die Zusammensetzung außerdem mindestens ein Mittel zur künstlichen Bräunung und/oder Braunfärbung der Haut umfasst.

27. Verwendung nach einem der Ansprüche 1 bis 26, **dadurch gekennzeichnet, dass** die Zusammensetzung außerdem mindestens einen Hilfsstoff umfasst, der aus Fettsubstanzen, organischen Lösungsmitteln, ionischen oder nicht-ionischen Verdickungsmitteln, Enthärtungsmitteln, Antioxidantien, Mitteln gegen freie Radikale, Trübungsmitteln, Stabilisatoren, Weichmachern, Silikonen, α-Hydroxysäuren, Antischaummitteln, Hydratisierungsmitteln, Vitaminen, Insektenabwehrmitteln, Parfums, Konservierungsmitteln, Tensiden, entzündungshemmenden Mitteln, Antagonisten von Substanz P, Füllstoffen, Polymeren, Treibmitteln, Alkalinisierungsmitteln oder Ansäuerungsmitteln, Färbemitteln ausgewählt ist.

28. Verwendung nach einem der Ansprüche 1 bis 27, **dadurch gekennzeichnet, dass** die Zusammensetzung eine Schutzzusammensetzung für die menschliche Epidermis oder eine Sonnenschutzzusammensetzung ist und dass sie in Form einer nicht-ionischen Vesikeldispersion, einer Emulsion, insbesondere einer Emulsion des Öl-in-Wasser-Typs, einer Creme, einer Milch, eines Gels, eines Cremegels, einer Suspension, einer Dispersion, eines Pulvers, eines festen Stiftes, eines Schaums oder eines Sprays vorliegt.

29. Verwendung nach einem der Ansprüche 1 bis 27, **dadurch gekennzeichnet, dass** die Zusammensetzung eine Zusammensetzung zum Schminken der Wimpern, der Augenbrauen oder der Haut ist und dass sie in fester oder pastöser, wasserfreier oder wässriger Form, in Form einer Emulsion, einer Suspension oder einer Dispersion vorliegt.

30. Verwendung nach einem der Ansprüche 1 bis 27, **dadurch gekennzeichnet, dass** die Zusammensetzung eine für den Schutz der Haare gegen ultraviolette Strahlen bestimmte Zusammensetzung ist und dass sie in Form eines Shampoos, einer Lotion, eines Gels, einer Emulsion, einer nicht-ionischen Vesikeldispersion vorliegt.

## Claims

1. Use of an amino-substituted 2-hydroxybenzophenone derivative of following formula (VIII): 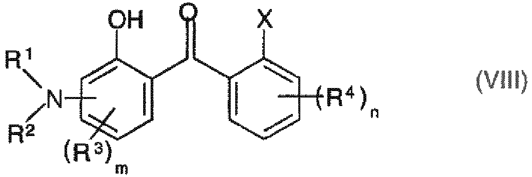 in which:
R¹ and R², which are identical or different, denote a hydrogen atom, a C₁-C₂₀ alkyl radical, a C₂-C₁₀ alkenyl radical, a C₃-C₁₀ cycloalkyl radical or a C₃-C₁₀ cycloalkenyl radical;
R¹ and R² can also form, with the nitrogen atom with which they are bonded, a 5- or 6-membered ring;
R³ and R⁴, which are identical or different, denote a C₁-C₂₀ alkyl radical, a C₂-C₁₀ alkenyl radical, a C₃-C₁₀ cycloalkyl radical, a C₃-C₁₀ cycloalkenyl radical, a C₁-C₁₂ alkoxy radical, a (C₁-C₂₀) alkoxycarbonyl radical, a C₁-C₁₂ alkylamino radical, a di(C₁-C₁₂)alkylamino radical, an aryl radical or a heteroaryl which is optionally substituted, or a water-solubilizing substituent chosen from a carboxylate group, a sulphonate group or an ammonium residue;
X denotes a hydrogen atom or a COOR⁵ or CONR⁶R⁷ group;
R⁵, R⁶ and R⁷, which are identical or different, denote a hydrogen atom, a C₁-C₂₀ alkyl radical, a C₂-C₁₀ alkenyl radical, a C₃-C₁₀ cycloalkyl radical, a C₃-C₁₀ cycloalkenyl radical, a -(YO)ₒ-Z group or an aryl group;
Y denotes -(CH₂)₂-, - (CH₂)₃-, -(CH₂)₄- or -CH-CH₃-CH₂-;
Z represents -CH₂-CH₃, -CH₂CH₂CH₃, -CH₂-CH₂-CH₂-CH₃ or -CH(CH₃) -CH₃;
m is an integer varying from 0 to 3;
n is an integer varying from 0 to 3;
o is an integer varying from 1 to 2;
for the manufacture of a cosmetic or dermatological composition for topical use, in particular for the photoprotection of the skin and hair, which comprises, in a cosmetically acceptable vehicle:
(a) at least one dibenzoylmethane derivative and
(b) at least one 1,3,5-triazine derivative which is photosensitive in the presence of a dibenzoylmethane derivative corresponding to the following formula (I): 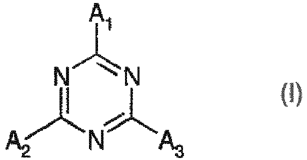
in which the A₁, A₂ and A₃ radicals, which are identical or different, are chosen from the groups of formulae (II): 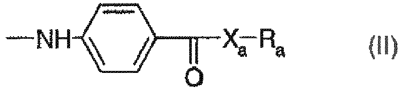 in which:
- Xₐ, which are identical or different, represent oxygen or the -NH- radical;
- Rₐ, which are identical or different, are chosen from: hydrogen; an alkali metal; an ammonium radical optionally substituted by one or more alkyl or hydroxyalkyl radicals; a linear or branched C₁-C₁₈ alkyl radical; a C₅-C₁₂ cycloalkyl radical optionally substituted by one or more C₁-C₄ alkyl radicals; a polyoxyethylenated radical comprising from 1 to 6 ethylene oxide units and for which the terminal OH group is methylated; and a radical of following formula (III), (IV) or (V): 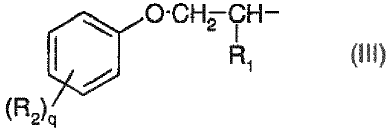 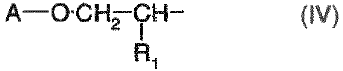 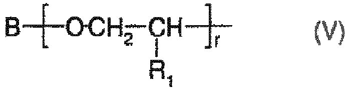
in which:
- R₁ is hydrogen or a methyl radical;
- R₂ is a C₁-C₉ alkyl radical;
- q is an integer ranging from 0 to 3;
- r is an integer ranging from 1 to 10;
- A is a C₄-C₈ alkyl radical or a C₅-C₈ cycloalkyl radical;
- B is chosen from: a linear or branched C₁-C₈ alkyl radical; a C₅-C₈ cycloalkyl radical; or an aryl radical optionally substituted by one or more C₁-C₄ alkyl radicals, and
C) at least the said amino-substituted 2-hydroxybenzophenone derivative,
the said composition not comprising p-methyl-benzylidenecamphor;
the ratio by weight of the 2-hydroxybenzophenone derivative to the dibenzoylmethane derivative being greater than 1,
for the purpose of improving, in the said composition, the stability to UV radiation of the said 1,3,5-triazine derivative.

2. Use according to Claim 1, **characterized in that** the 1,3,5-triazine derivative is chosen from those where the A₁, A₂ and A₃ radicals are of formula (II) and exhibiting all of the following characteristics:
- one or two Xₐ-Rₐ groups represent the -NH-Rₐ radical with Rₐ chosen from: a linear or branched C₁-C₁₈ alkyl radical; a C₅-C₁₂ cycloalkyl radical optionally substituted with one or more C₁-C₄ alkyl radicals; or a radical of formula (III), (IV) or (V) above in which:
- B is a C₁-C₄ alkyl radical;
- R₂ is the methyl radical;
the other Xₐ-Rₐ group or the other two Xₐ-Rₐ groups being the -O-Rₐ radical with Rₐ, which are identical or different, chosen from: hydrogen; an alkali metal; an ammonium radical optionally substituted with one or more alkyl or hydroxyalkyl radicals; a linear or branched C₁-C₁₈ alkyl radical; a C₅-C₁₂ cycloalkyl radical optionally substituted with one or more C₁-C₄ alkyl radicals; or a radical of formula (III), (IV) or (V) above in which:
- B is a C₁-C₄ alkyl radical;
- R₂ is the methyl radical.

3. Use according to Claim 2, **characterized in that** the 1,3,5-triazine derivative is that corresponding to the following formula: 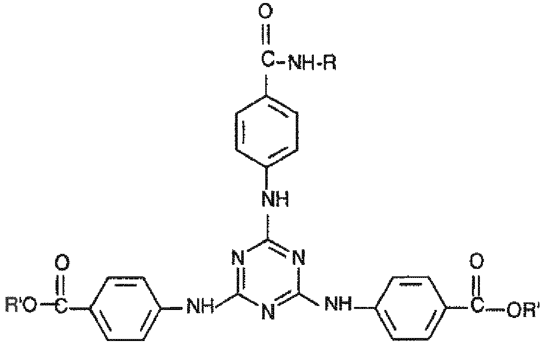 in which R' denotes a 2-ethylhexyl radical and R denotes a tert-butyl radical.

4. Use according to Claim 1, **characterized in that** the 1,3,5-triazine derivative corresponding to the formula (I) is chosen from those where the A₁, A₂ and A₃ radicals are of formula (II) and exhibit the following characteristics:
- Xₐ are identical and represent oxygen;
- Rₐ, identical or different and represent a C₆-C₁₂ alkyl radical or a polyoxyethylenated radical comprising from 1 to 6 ethylene oxide units and for which the terminal OH group is methylated.

5. Use according to Claim 4, **characterized in that** the 1,3,5-triazine derivative is that corresponding to the following formula: 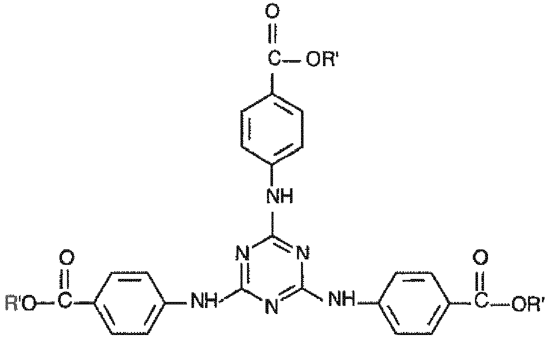 in which R' denotes a 2-ethylhexyl radical.

6. Use according to any one of Claims 1 to 5, **characterized in that** the 1,3,5-triazine derivative is present in the composition at contents ranging from 0.5 to 15% by weight and preferably from 1% to 10% by weight with respect to the total weight of the composition.

7. Use according to any one of Claims 1 to 6, **characterized in that** the dibenzoylmethane derivative is chosen from:
2-methyldibenzoylmethane
- 4-methyldibenzoylmethane
- 4-isopropyldibenzaylmethane
- 4-tert-butyldibenzoylmethane
- 2,4-dimethyldibenzoylmethane
- 2,5-dimethyldibenzoylmethane
- 4,4'-diisopropyldibenzoylmethane
- 4,4'-dimethoxydibenzoylmethane
- 4-tert-butyl-4'-methoxydibenzoylmethane
- 2-methyl-5-isopropyl-4'-methoxydibenzoylmethane
- 2-methyl-5-tert-butyl-4'-methoxydibenzoylmethane
- 2,4-dimethyl-4'-methoxydibenzoylmethane
- 2,6-dimethyl-4-tert-butyl-4'-methoxydibenzoylmethane.

8. Use according to Claim 7, **characterized in that** the dibenzoylmethane derivative is 4-tert-butyl-4'-methoxydibenzoylmethane.

9. Use according to Claim 7, **characterized in that** the dibenzoylmethane derivative is 4-isopropyldibenzoylmethane.

10. Use according to any one of Claims 1 to 9, where the dibenzoylmethane derivative is present in the composition at contents ranging from 0.5 to 15% by weight and preferably from 1% to 10% by weight with respect to the total weight of the composition.

11. Use according to any one of Claims 1 to 10, where the compound or compounds of formula (VIII) are chosen from those of following formula (VIIIa): 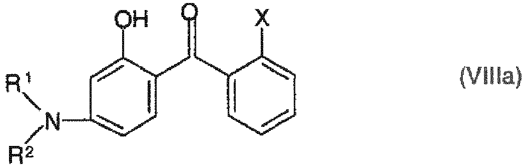 in which:
R¹ and R², which are identical or different, denote a hydrogen atom or a C₁-C₁₂ alkyl radical or form, with the nitrogen atom with which they are bonded, a 5- or 6-membered ring;
X denotes COOR⁵ or CONR⁶R⁷ ;
R⁵ denotes a hydrogen atom, a C₁-C₁₂ alkyl radical or a C₃-C₆ cycloalkyl radical;
R⁶ and R⁷, which are identical or different, denote a hydrogen atom, a C₁-C₁₂ alkyl radical or a C₅-C₆ cycloalkyl radical.

12. Use according to Claim 11, where the compounds of formula (VIIIa) are those for which:
R¹ and R², which are identical or different, denote a C₁-C₄ alkyl radical and more particularly ethyl;
R⁵ denotes a C₃-C₈ alkyl radical;
R⁶ and R⁷, which are identical or different, denote a C₁-C₈ alkyl radical.

13. Use according to any one of Claims 1 to 10, where the compound or compounds of formula (VIII) are chosen from those of following formula (VIIIb): 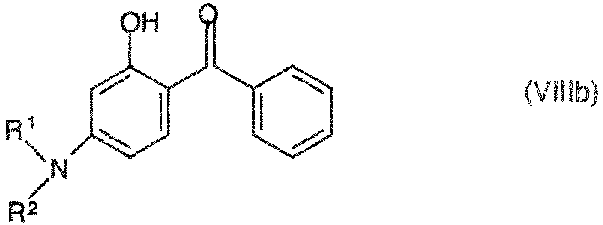 in which:
R¹ and R², which are identical or different, denote a C₁-C₁₂ alkyl radical or form, with the nitrogen atom with which they are bonded, a 5- or 6-membered ring.

14. Use according to Claim 13, where the compound of formula (VIIIb) is chosen from:
- 4-diethylamino-2-hydroxyphenyl phenyl ketone,
- 4-pyrrolidino-2-hydroxyphenyl phenyl ketone.

15. Use according to any one of Claims 1 to 10, where the compound or compounds of formula (VIII) are chosen from those of following formula (VIIIc): 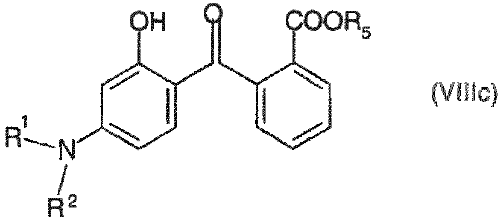 in which:
R¹ and R², which are identical or different, denote a hydrogen atom or a C₁-C₈ alkyl radical or form, with the nitrogen atom with which they are bonded, a 5- or 6-membered ring;
R⁵ denotes a hydrogen atom, a C₁-C₁₂ alkyl radical or a C₃-C₆ cycloalkyl radical.

16. Use according to Claim 15, where the compound of formula (VIIIc) is chosen from:
- 2-(4-pyrrolidino-2-hydroxybenzoyl)benzoate
- methyl 2-(4-diethylamino-2-hydroxybenzoyl)benzoate
- 2-Ethylhexyl 2-(4-diethylamino-2-hydroxybenzoyl)-benzoate
- cyclohexyl 2-(4-diethylamino-2-hydroxybenzoyl)-benzoate
- n-hexyl 2-(4-diethylamino-2-hydroxybenzoyl)benzoate
- 2-(4-dibutylamino-2-hydroxybenzoyl)benzoate
- methyl 2-(4-dibutylamino-2-hydroxybenzoyl)benzoate
- isobutyl 2-(4-dibutylamino-2-hydroxybenzoyl)benzoate,

17. Use according to Claim 16, where the compound of formula (VIIIc) is n-hexyl 2-(4-diethylamino-2-hydroxybenzoyl)benzoate.

18. Use according to any one of Claims 1 to 17, in which the amino-substituted 2-hydroxybenzophenone derivative or derivatives of formula (VIII) are present in proportions ranging from 0.1% to 15% by weight with respect to the total weight of the composition.

19. Use according to Claim 18, in which the amino-substituted 2-hydroxybenzophenone derivative or derivatives of formula (VIII) are present in the composition in proportions ranging from 1 to 10% by weight with respect to the total weight of the composition.

20. Use according to Claim 19, in which the amino-substituted 2-hydroxybenzophenone derivative or derivatives of formula (VIII) are present in the composition in proportions ranging from 2 to 8% by weight with respect to the total weight of the composition.

21. Use according to any one of Claims 1 to 20, **characterized in that** the composition additionally comprises other organic screening agents which are active in the UV-A and/or UV-B regions.

22. Use according to Claim 21, where the additional organic UV screening agent or agents are chosen from anthranilates; salicylic derivatives; cinnamic derivatives; camphor derivatives, other than p-methylbenzylidenecamphor; benzophenone derivatives, other than those of formula (VIII); β,β-diphenylacrylate derivatives; benzotriazole derivatives; non-photosensitive triazine derivatives; benzalmalonate derivatives; benzimidazole derivatives; imidazolines; bisbenzoazolyl derivatives; p-aminobenzoic acid (PABA) derivatives; methylenebis(hydroxy-phenylbenzotriazole) derivatives; screening polymers and silicones; dimers derived from α-alkylstyrene; 4,4-diarylbutadienes and their mixtures.

23. Use according to Claim 22, **characterized in that** the organic UV screening agent or agents are chosen from the following compounds:
- Ethylhexyl Salicylate,
- Ethylhexyl Methoxycinnamate,
- Octocrylene,
- Phenylbenzimidazole Sulfonic Acid,
- Benzophenone-3,
- Benzophenone-4,
- Benzophenone-5,
- 4-Methylbenzylidene camphor,
- Terephthalylidene Dicamphor Sulfonic Acid,
- Disodium Phenyl Dibenzimidazole Tetrasulfonate,
- 2,4,6-Tris(diisobutyl 4'-aminobenzalmalonate)-s-triazine,
- Anisotriazine,
- Methylenebis(benzotriazolyltetramethylbutylphenol),
- Drometrizole Trisiloxane,
- -1,1-dicarboxy(2,2'-dimethyl-propyl)-4,4-diphenylbutadiene
and their mixtures.

24. Use according to any one of Claims 1 to 20, **characterized in that** the composition additionally comprises coated or uncoated metal oxide pigments or nanopigments.

25. Use according to Claim 24, **characterized in that** the said pigments or nanopigments are chosen from coated or uncoated titanium oxide, zinc oxide, iron oxide, zirconium oxide or cerium oxide, and their mixtures.

26. Use according to any one of Claims 1 to 25, **characterized in that** the composition additionally comprises at least one agent for the artificial tanning and/or browning of the skin.

27. Use according to any one of Claims 1 to 26, **characterized in that** the composition additionally comprises at least one adjuvant chosen from fatty substances, organic solvents, ionic or nonionic thickeners, softeners, antioxidants, agents for combating free radicals, opacifiers, stabilizers, emollients, silicones, α-hydroxy acids, antifoaming agents, moisturizing agents, vitamins, insect repellents, fragrances, preservatives, surfactants, anti-inflammatories, substance P antagonists, fillers, polymers, propellants, basifying or acidifying agents, or colorants.

28. Use according to any one of Claims 1 to 27, **characterized in that** the composition is a protective composition for the human epidermis or an antisun composition and **in that** it is provided in the form of a nonionic vesicular dispersion, of an emulsion, in particular of an emulsion of oil-in-water type, of a cream, of a milk, of a gel, of a cream gel, of a suspension, of a dispersion, of a powder, of a solid tube, of a foam or of a spray.

29. Use according to any one of Claims 1 to 27, **characterized in that** the composition is a composition for making up the eyelashes, eyebrows or skin and **in that** it is provided in the anhydrous or aqueous, pasty or solid form, in the form of an emulsion, of a suspension or of a dispersion.

30. Use according to any one of Claims 1 to 27, **characterized in that** the composition is a composition intended for the protection of the hair against ultraviolet rays and **in that** it is provided in the form of a shampoo, of a lotion, of a gel, of an emulsion or of a nonionic vesicular dispersion.
